# EUROPEAN PATENT SPECIFICATION

(11) **EP 3 726 197 B1**
(45) Date of publication and mention of the grant of the patent: **25.01.2023**
(21) Application number: 18889890.2
(22) Date of filing: 12.11.2018
(51) Int. Cl.: G01N 21/01, A61B 5/023, G01N 21/17, G01N 21/359, H01L 31/12, H01S 5/02, A61B 5/00, A61B 5/026, G01N 21/47, G01N 21/49, G01N 33/02, A61B 5/024

(54) **OPTICAL SENSING DEVICE**
OPTISCHE SENSORVORRICHTUNG
DISPOSITIF DE DÉTECTION OPTIQUE

(30) Priority: 13.12.2017 JP 2017238286
(43) Date of publication of application: 21.10.2020
(73) Proprietor: Panasonic Intellectual Property Management Co., Ltd., Osaka-shi, Osaka 540-6207 (JP)
(72) Inventor: SHIONO Teruhiro, Osaka-shi Osaka 540-6207 (JP); ANDO, Takamasa, Osaka-shi Osaka 540-6207 (JP)
(74) Representative: Grünecker Patent- und Rechtsanwälte PartG mbB
(86) International application number: PCT/JP2018/041799
(87) International publication number: WO 2019/116803

(56) References cited:
- WO-A1-2014/100378
- WO-A1-2015/117241
- WO-A1-2016/164894
- JP-A- H0 556 982
- JP-A- H10 185 815
- JP-A- 2013 505 468
- JP-A- 2015 092 151
- JP-A- 2016 093 280
- JP-A- 2016 128 802
- JP-A- 2016 130 669
- JP-A- 2017 106 869
- JP-A- 2017 144 225
- JP-A- 2017 185 200
- JP-A- 2017 504 836
- JP-A- 2018 146 525
- JP-A- 2018 183 579
- US-A1- 2012 257 034
- US-A1- 2015 241 342
- US-A1- 2016 373 652
- US-A1- 2017 079 530
- US-A1- 2017 142 314
- US-A1- 2017 289 469

## Description

### Technical Field

The present invention relates to an optical sensing apparatus.

### Background Art

In recent years, an optical sensing apparatus has been used to obtain, in a non-contact manner, useful information on the interior of a physical object such as a living body or food. The optical sensing apparatus includes a laser device and a photodetector. The laser device irradiates the physical object with laser light. The photodetector detects reflected scattered light coming out from a surface of the physical object after being multiply scattered in the interior of the physical object.

In a case where the physical object is a living body, light emitted from the laser device penetrates inside of the living body through the skin. After that, the reflected scattered light coming out from the skin contains biometric information such as a condition of the blood by having passed through a blood vessel or the like. By detecting the reflected scattered light, information such as the pulse, blood pressure, blood flow, and oxygen saturation can be obtained. These pieces of information can be used in a physical examination.

For example, laser light with a near-infrared wavelength of 700 to 950 nm has the property of passing through body tissue such as muscles, fat, and bones with a comparatively high transmissivity. Meanwhile, laser light in a near-infrared wavelength region also have the property of being easily absorbed into oxyhemoglobin (HbO₂) and deoxyhemoglobin (Hb) in the blood. Accordingly, biometric information measurements commonly involve the use of laser devices that irradiate physical objects with laser light in a near-infrared wavelength region.

Through the use of such a laser device, information about intracerebral blood flow can be acquired by irradiating the forehead with laser light and detecting reflected scattered light. For example, the amount of change in intracerebral blood flow and the respective amounts of change in concentration of oxyhemoglobin and deoxyhemoglobin in the blood can be measured. A state of brain activity can be estimated on the basis of the amount of change in blood flow, an oxygen state of hemoglobin, or the like.

Further, information such as the freshness and sugar content of food can be obtained in a non-destructive manner by irradiating the food with laser light and detecting reflected scattered light from inside of the food.

PTL 1 discloses an optical cerebral function measuring apparatus that measures a cerebral function in a non-contact manner. PTL 2 discloses an imaging system that performs a time-resolved measurement with improvement in S/N ratio of signal light returning from a deep place in body tissue.

Further, PTLs 3 and 4 disclose a laser device that enlarges an apparent light source size by irradiating a scatterer with laser light and using scattered light thus spread.

### Citation List

### Patent Literature

PTL 1: Japanese Unexamined Patent Application Publication No. 2003-337102
PTL 2: Japanese Unexamined Patent Application Publication No. 2007-260123
PTL 3: WO 2003-077389
PTL 4: Japanese Unexamined Patent Application Publication No. 2012-169375

US 2017/0289469 discloses an optical sensing apparatus comprising a laser device including: a light source that emits laser light; and a diffusing member having a diffusing surface that crosses an optical path of the laser light, the diffusing member refracting or diffracting the laser light to make the laser light lower in intensity in a first portion including a center of a cross-section of the laser light that crosses the optical path, and make the laser light higher in intensity in a second portion of the laser light that surrounds the first portion in the cross-section, and enlarge a beam diameter of the laser light. The laser device is configured to irradiate a physical object with the laser light.

### Summary of Invention

### Technical Problem

The present disclosure provides an optical sensing apparatus that can improve the safety of laser products and bring about improvement in S/N ratio. Solution to Problem

An optical sensing apparatus according to the present invention includes the features of claim 1.

### Advantageous Effects of Invention

An aspect of the present disclosure makes it possible to provide an optical sensing apparatus that can improve the safety of laser products and bring about improvement in S/N ratio.

### Brief Description of Drawings

[Fig. 1] Fig. 1 is a schematic view for explaining a configuration of a laser device and how a physical object is irradiated with light emitted from the laser device.
[Fig. 2A] Fig. 2A is a plan view schematically showing a structure of a diffusing member of the laser device.
[Fig. 2B] Fig. 2B is a cross-sectional view schematically showing the structure of the diffusing member of the laser device.
[Fig. 3A] Fig. 3A is a diagram schematically showing a shape of light on a diffusing surface of the laser device.
[Fig. 3B] Fig. 3B is a diagram schematically showing a light intensity distribution of the light on the diffusing surface of the laser device.
[Fig. 3C] Fig. 3C is a diagram schematically showing a shape of light on a screen of the laser device.
[Fig. 3D] Fig. 3D is a diagram schematically showing a light intensity distribution of the light on the screen of the laser device.
[Fig. 3E] Fig. 3E is a diagram schematically showing a shape of light on a surface of the physical object.
[Fig. 3F] Fig. 3F is a diagram schematically showing a light intensity distribution of the light on the surface of the physical object.
[Fig. 3G] Fig. 3G is an example of a diagram showing a light intensity distribution on the diffusing surface.
[Fig. 3H] Fig. 3H is an example of a diagram showing a light intensity distribution on the screen.
[Fig. 4] Fig. 4 is a schematic view for explaining a configuration of an optical sensing apparatus and how a biometric measurement is carried out.
[Fig. 5] Fig. 5 is a diagram schematically showing an internal configuration of a control circuit and a photodetector of the optical sensing apparatus and the flow of signals.
[Fig. 6A] Fig. 6A is a diagram showing an example of a time distribution of a single optical pulse serving as emitted light.
[Fig. 6B] Fig. 6B is a diagram showing total optical power (solid line) detected in a stationary state, an amount of change (dotted line) in optical power corresponding to an amount of change in cerebral blood flow, and a time distribution of degrees of modulation (dot-and-dash line).
[Fig. 7] Fig. 7 is a diagram schematically showing a time distribution of optical pulses emitted from the laser device, a time distribution of optical power detected by the photodetector of the optical sensing apparatus, and timings and charge storage of an electronic shutter.
[Fig. 8A] Fig. 8A is a front view showing changes in blood flow that are present inside the physical object.
[Fig. 8B] Fig. 8B is a side cross-sectional view showing changes in blood flow that are present in the physical object.
[Fig. 9A] Fig. 9A is a diagram schematically showing changes in blood flow in the interior of the physical object.
[Fig. 9B] Fig. 9B is a diagram schematically showing changes in blood flow in the interior of the physical object as image-corrected by image computations.
[Fig. 10] Fig. 10 is a schematic view for explaining a configuration of a laser device and how a physical object is irradiated with light emitted from the laser device.
[Fig. 11] Fig. 11 is a schematic view for explaining a configuration of a laser device and how a physical object is irradiated with light emitted from the laser device.
[Fig. 12] Fig. 12 is a schematic view for explaining a configuration of a laser device and how a physical object is irradiated with light emitted from the laser device.
[Fig. 13] Fig. 13 is a schematic view for explaining a configuration of a laser device and how a physical object is irradiated with light emitted from the laser device.
[Fig. 14] Fig. 14 is a diagram schematically showing an internal configuration of a control circuit and a photodetector of an optical sensing apparatus and the flow of signals.
[Fig. 15] Fig. 15 is a diagram schematically showing a time distribution of optical pulses emitted from the laser device, a time distribution of optical power detected by the photodetector of the optical sensing apparatus, and timings and charge storage of an electronic shutter.
[Fig. 16] Fig. 16 is a schematic view for explaining a configuration of a conventional laser device and how a physical object is irradiated with light emitted from the laser device.
[Fig. 17A] Fig. 17A is a diagram schematically showing a time distribution of optical power of an optical pulse emitted from a light source of the conventional laser device.
[Fig. 17B] Fig. 17B is a diagram schematically showing a time distribution of optical power of light with which a physical object was irradiated by the conventional laser device.

### Description of Embodiments

First, prior to a description of embodiments of the present disclosure, underlying knowledge forming the basis of an optical sensing apparatus according to the present disclosure is described.

PTL 1 discloses an optical cerebral function measuring apparatus that measures a cerebral function in a non-contact manner by irradiating the forehead of a subject with laser light. In PTL 1, in irradiating the forehead of a subject with the laser light, the subject wears a light-shielding member such as a sleeping mask for protection of the eyes.

PTL 2 discloses an imaging system that performs a time-resolved measurement with improvement in S/N ratio of signal light returning from a deep place in body tissue. In PTL 2, optical pulses are used as illuminating light to delay an imaging timing, whereby intense noise light that returns temporally early is not imaged. This brings about improvement in S/N ratio of the signal light. In PTL 2, an endoscopic apparatus is used to observe blood flow information on a blood vessel buried in body tissue covered with visceral fat.

PTL 3 discloses a laser device that enlarges an apparent light source size by using scattered light coming out spread by multiple reflection that occurs inside a scatterer covering an exit end of a semiconductor laser.

PTL 4 discloses an automotive headlight unit. In PTL 4, a scattering part containing TiO₂ microparticles and a phosphor is irradiated with laser light. Wavelength-converted fluorescence is produced by the phosphor being excited by scattered light spread by multiple reflection within the scattering part. The fluorescence and the scattered light coming out spread turns into parallel light through a reflecting mirror. Light obtained by the parallel light passing through a diffusing member is emitted from the automotive headlight unit.

In a case where the eye of a subject are fitted with a light-shielding member as in the case of PTL 1, a task that is executed in performing a brain measurement is limited to the audio. This results in a low degree of freedom in measurement. In a case where a measurement is carried out without a light-shielding member fitted on the eyes, a laser product that meets the safety standards of the eyes is used. The laser device is limited in maximum permissible exposure (MPE) and accessible emission limit (AEL) for Class 1 provided for by the safety standards of laser products (JIS C 6802). Therefore, the power of the laser light is set low to meet MPE and AEL for Class 1. Parallel light such as that of PTL 1 easily affects the eyes in particular. In a case where the laser light is a continuum of emissions with a wavelength of, for example, 850 nm, the maximum value of AEL is 0.78 mW, which is very small. In a case where such laser light is used, the S/N ratio of a brain measurement becomes very poor.

In PTLs 3 and 4, an apparent light source size is enlarged by irradiating a scatterer with laser light and using scattered light coming out spread and fluorescence. The term "apparent light source size" here means the size of a currently light-emitting region as viewed by a subject. The scatterer, which is a radiation source of diverging light coming out spread, can be considered as an extended source. In general, diverging light provides more enhanced safety than parallel light. AEL and MPE for Class 1 are greater in the case of diverging light than in the case of parallel light. In the case of diverging light from an extended source, AEL and MPE for Class 1 are fixed at a minimum distance, included in an eye-focusing range, at which the eyes are brought to a focus under the most dangerous conditions.

For example, in the case of an apparent light source size of larger than 10 mm, the eyes are brought to a focus at a distance of 100 mm or longer from a light source. Accordingly, with the eyes having an aperture diameter of 7 mm in the case of a distance of 100 mm, AEL and MPE are determined by optical power within the range of an aperture diameter of 7 mm. In the case of an apparent light source size of smaller than 10 mm, the smaller a light source is in size, the shorter the distance from light source at which the eyes are brought to a focus becomes. For example, in the case of a light source size of 1.5 mm, the minimum distance at which the eyes are brought to a focus is 39. 3 mm. In the case of a light source size of 3 mm, the minimum distance at which the eyes are brought to a focus is 55.2 mm. Accordingly, in the case of a light source size of smaller than 10 mm, the smaller a light source is in size, the shorter the minimum distance at which the eyes are brought to a focus is. Accordingly, determining the values of AEL and MPE by optical power within the range of an aperture diameter of 7 mm at that minimum distance results in decreases in the values of AEL and MPE.

When an apparent light source size is smaller than 10 mm, AEL and MPE for Class 1 can be increased in proportion to the square of the apparent light source size. This makes it possible to irradiate a physical object with increased power while maintaining safety.

Assume a case where the S/N ratio of an optical sensing apparatus is improved by irradiating a physical object with laser light from the laser device of PTL 3 or 4 (hereinafter referred to as "conventional laser device") and performing a time-resolved measurement with the optical sensing apparatus of PTL 2 (hereinafter referred to as "conventional optical sensing apparatus"). The inventors found that in the conventional optical sensing apparatus, multiple scattering inside a scatterer that has an effect of spreading laser light reduces the S/N ratio of a time-resolved measurement. The following gives a detailed description.

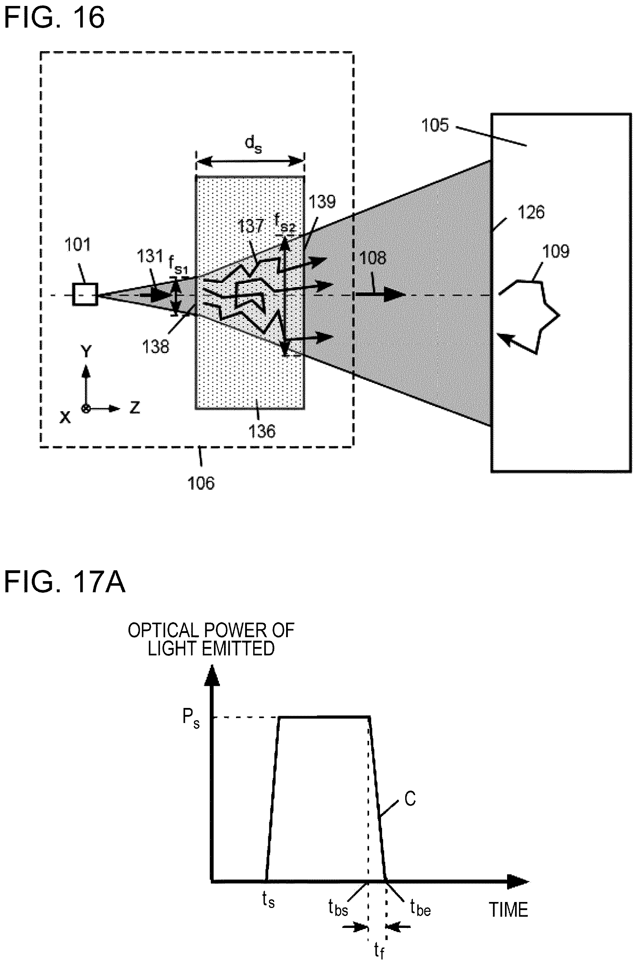

Fig. 16 is a schematic view for explaining a configuration of a conventional laser device 106 and how a physical object is irradiated with light emitted from the laser device 106.

The conventional laser device 106 includes a light source 101 and a scatterer 136 having a thickness dₛ. The conventional optical sensing apparatus includes the laser device 106 and a photodetector (not illustrated).

In the example shown in Fig. 16, emitted light 131 emitted from the light source 101 falls on the scatterer 136. Light 138 on a surface of incidence of the scatterer 136 has an optical beam diameter fₛ₁. The incident light 138 is repeatedly multiply scattered in the interior of the scatterer 136. Scattered light 137 is emitted spread from the scatterer 136. Light 139 on a surface of emergence of the scatterer 136 has an optical beam diameter fₛ₂. The optical beam diameter fₛ₂ is larger than the optical beam diameter fₛ₁.

The apparent light source size is enlarged from fₛ₁ to fₛ₂ by the scatterer 136. As a result, AEL and MPE for Class 1 can be increased by the size of the square of a scale of enlargement (fₛ₂/fₛ₁). Using a thicker scatterer 136 can make the scale of enlargement larger and therefore can make AEL and MPE for Class 1 greater. However, a thicker scatterer 136 reduces the intensity of scattered light that is emitted from the scatterer 136.

In the example shown in Fig. 16, irradiating light 108, which is scattered light, gets further spread. Irradiating a physical object 105 with spread light 126 turns the light 126 into internally-scattered light 109. The internally-scattered light 109 is emitted outward as reflected scattered light (not illustrated) containing internal information on the physical object and detected by a photodetector (not illustrated).

Fig. 17A is a diagram schematically showing a time distribution of optical power of an optical pulse emitted from a light source of the conventional laser device. In a case where a short optical pulse of, for example, approximately 1 to 20 ns has been emitted from the light source 101, a time distribution of optical power of light emitted from the light source 101 is for example shaped as shown in Fig. 17A. During a period from time tₛ to t_{be}, a trapezoidal optical pulse having a maximum optical power P_{S} is emitted from the light source 101. A period of time from time t_{bs} to t_{be} during which the optical power falls is a falling period of this optical pulse, and t_{f} (= t_{be} - t_{bs}) is a falling time.

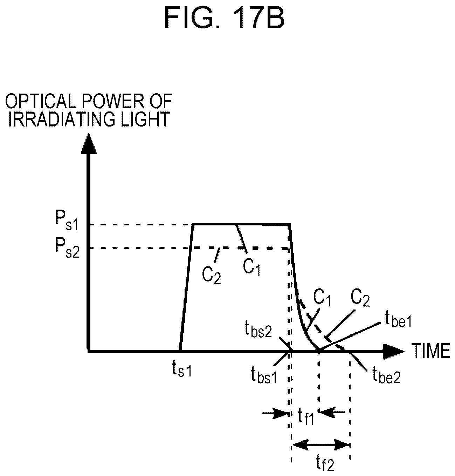

Fig. 17B is a diagram schematically showing a time distribution of optical power of light with which a physical object was irradiated by the conventional laser device in a case where an optical pulse has passed through a thin scatterer or a thick scatterer.

In the example shown in Fig. 16, the point of intersection between an optical path indicated by a dot-and-dash line and the surface of emergence of the scatterer 136 is the central part of the surface of emergence of the scatterer 136. A time distribution of optical power of the light 139 in the central part of the surface of emergence of the scatterer 136 is for example shaped as shown in Fig. 17B. The light 139 emitted from the scatterer 136 turns into the irradiating light 108. The thickness dₛ assumes two different types of scatterer. A time distribution of optical power in the case of a thick scatterer is represented by a dotted line, and a distribution of optical power in the case of a thin scatterer is represented by a solid line.

In the case of a thick scatterer, the irradiating light 108 is present during a period from time tₛ₁ to t_{be2} and has a maximum optical power P_{S2}. The irradiating light 108 is emitted as a trapezoidal optical pulse C₂ whose lower slope leaves a trail in a back-end region in the second half of a falling period. On the other hand, in the case of a thin scatterer, the irradiating light 108 is present during a period from time tₛ₁ to t_{be1} and has a maximum optical power P_{S1}. The irradiating light 108 is emitted as a trapezoidal optical pulse C₁ whose lower slope leaves a trail in a back-end region in the second half of a falling period. A thicker scatterer is longer in falling period length and smaller in optical power than a thinner scatterer. The falling time in the case of a thick scatterer is t_{f2}, and the falling time in the case of a thin scatterer is t_{f1}. The falling time t_{f2} in the case of a thick scatterer is longer than the falling time t_{f1} in the case of a thin scatterer.

The term "scattered light" refers to light that becomes spread by a change in direction of propagation of light by microparticles contained in the scatterer 136. As shown in Fig. 16, the scattered light 137 gets spread while being repeatedly multiply scattered while taking a zigzag optical path or changing optical paths, for example, from back to front or from front to back.

In the example shown in Fig. 16, all of the light 139 on the surface of emergence of the scatterer 136 is scattered light. Accordingly, in a case where a short optical pulse has been emitted from the light source 101, times of arrival of scattered light at the surface of emergence of the scatterer 136 are not identical but vary. A thicker scatterer 136 leads to a larger degree of multiple scattering. This results in a greater temporal variation in the light 139 and results in a longer falling time. Therefore, as shown in Fig. 17B, a thicker scatterer leads to a longer falling time of irradiating light.

The inventors studied use of the conventional optical sensing apparatus to illuminate a physical object with light with a long falling time that has passed through a scatterer and to perform a time-resolved measurement by detecting reflected scattered light from the physical object. As a result of their study, the inventors found that a thicker scatterer leads to not only a fall in irradiating power due to a great loss of light but also a longer falling time and that a longer falling time leads to a reduction in S/N ratio of a detected signal.

On the basis of the foregoing findings, the inventors conceived a laser device and an optical sensing apparatus including the same.

In the present disclosure, all or a part of any of circuit, unit, device, part or portion, or any of functional blocks in the block diagrams may be implemented as one or more of electronic circuits including, but not limited to, a semiconductor device, a semiconductor integrated circuit (IC) or an LSI. The LSI or IC can be integrated into one chip, or also can be a combination of plural chips. For example, functional blocks other than a memory may be integrated into one chip. The name used here is LSI or IC, but it may also be called system LSI, VLSI (very large scale integration), or ULSI (ultra large scale integration) depending on the degree of integration. A Field Programmable Gate Array (FPGA) that can be programmed after manufacturing an LSI or a reconfigurable logic device that allows reconfiguration of the connection or setup of circuit cells inside the LSI can be used for the same purpose.

Further, it is also possible that all or a part of the functions or operations of the circuit, unit, device, part or portion are implemented by executing software. In such a case, the software is recorded on one or more non-transitory recording media such as a ROM, an optical disk or a hard disk drive, and when the software is executed by a processor, the software causes the processor together with peripheral devices to execute the functions specified in the software. A system or apparatus may include such one or more non-transitory recording media on which the software is recorded and a processor together with necessary hardware devices such as an interface.

In the following, embodiments of the present disclosure are more specifically described. Note, however, that an unnecessarily detailed description may be omitted. For example, a detailed description of a matter that has already been well known and a repeated description of substantially identical configurations may be omitted. This is intended to prevent the following description from becoming unnecessarily redundant and facilitate understanding of persons skilled in the art. It should be noted that the inventors provide the accompanying drawings and the following description so that persons skilled in the art can sufficiently understand the present disclosure, and do not intend to thereby limit the subject matters recited in the claims. In the following description, identical or similar constituent elements are given the same reference signs.

In the following, embodiments are described with reference to the drawings.

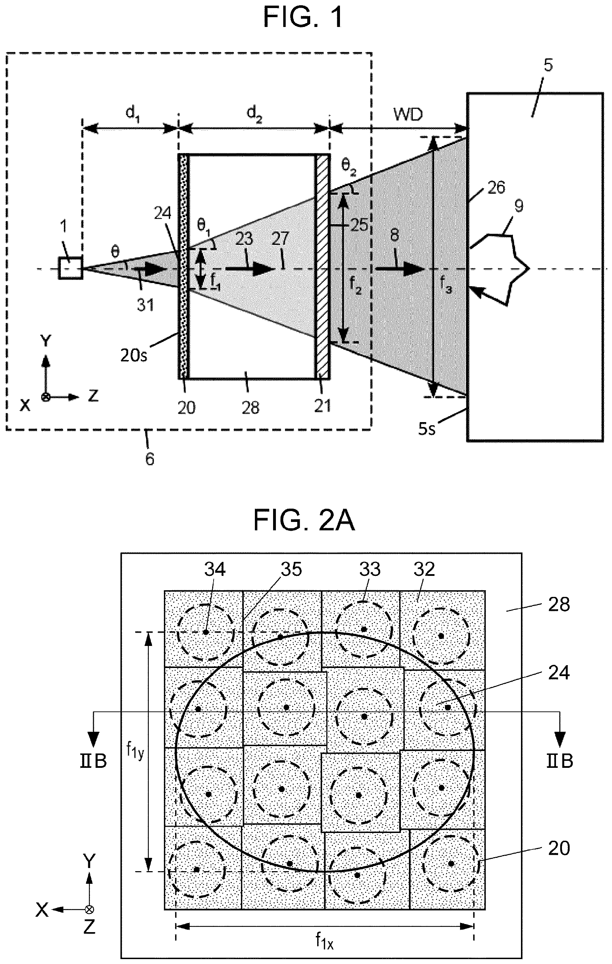

First, a laser device according to the present disclosure is described in detail with reference to Figs. 1 to 3C. Figs. 1 to 3C show XYZ coordinates whose X, Y, and Z directions are orthogonal to one another.

Fig. 1 is a schematic view for explaining a configuration of a laser device 6 of the present disclosure and how a physical object 5 is irradiated with light 8 emitted from the laser device 6.

The laser device 6 includes a laser light source 1, a diffusing member 20, and a screen 21.

The laser light source 1 is for example a semiconductor laser that repeatedly emits optical pulses. The laser light source 1 emits light of, for example, not shorter than 650 nm to not longer than 950 nm. This wavelength range is included in a wavelength range of red to near infrared radiation. The aforementioned wavelength range is called "biological window" and known to be low in absorptance in the body. The laser light source 1 is described as one that emits light falling within the aforementioned wavelength range, but light falling within another wavelength range may be used. The term "light" as used herein means not only visible light but also infrared radiation.

In a visible light region of shorter than 650 nm, absorption by hemoglobin in the blood is high, and in a wavelength range of longer than 950 nm, absorbance by water is high. Meanwhile, in a wavelength range of not shorter than 650 nm to not longer than 950 nm, the absorption coefficients of hemoglobin and water are comparatively low and the scattering coefficients of hemoglobin and water are comparatively high. Accordingly, light falling within this wavelength range is subjected to strong scattering after entering the body and returns to the body surface. This makes it possible to efficiently acquire information on the interior of the body. Therefore, the present example mainly uses light falling within this wavelength range.

The diffusing member 20 has a diffusing surface 20s that crosses the optical path of light 31 emitted from the laser light source 1. The diffusing member 20 refracts or diffracts light to make the light 31 lower in intensity in a central part serving as a first portion including the center of a cross-section of laser light that crosses the optical path and make the light 31 higher in intensity in a peripheral part serving as a second portion of the laser light that surrounds the first portion in the cross-section. For example, in a case where light having a Gaussian distribution of intensity has fallen on the diffusing member 20, the light is converted into light having an approximately-flat distribution of intensity and emitted from the diffusing member 20. A specific example of a configuration of the diffusing member 20 will be described later.

The screen 21 crosses an optical path 27 of light 23 having passed through the diffusing member 20. On a surface of the screen 21, there appears a spot of light having passed through the screen 21 or light reflected by the screen 21. The spot of light can be construed as being an apparent light source. The term "screen" as used herein refers to a member that does not have a function of greatly converting an intensity distribution of light and reflects the apparent light source. The screen 21 enlarges the apparent light source size. In the absence of the screen 21, the apparent light source size is the beam diameter f₁ of light 24 on the diffusing surface 20s. In the presence of the screen 21, the apparent light source size is the beam diameter f₂ of light 25 on the screen 21. The beam diameter f₂ of the light 25 on the screen 21 is larger than the beam diameter f₁ of the light 24 on the diffusing surface 20s. The apparent light source size on the screen 21 may be herein sometimes referred to as "light source size of the laser device 6". A specific example of a configuration of the screen 21 will be describer later.

In the example shown in Fig. 1, it is assumed that d₁ is the distance from the laser light source 1 to the diffusing member 20, that d₂ is the distance from the diffusing member 20 to the screen 21, and that WD is the distance from the screen 21 to the physical object 5. The light 31, which is emitted light, the light 23, which is deflected light or diffracted light emitted from the diffusing member 20, and the light 8, which is irradiating light, are each diverging light oriented in the Z direction on the optical path. The diffusing member 20 and the screen 21 are each placed orthogonal to the optical path 27 and parallel to the Y direction. An example is illustrated in which the optical path of the light 23 emitted from the diffusing member 20 is parallel to the Z direction. Without being bound by such a configuration, the optical path of the light 23 may be set at a slant from the Z direction. In that case, the screen 21 may be placed at a tilt from the Y direction so that the screen 21 is perpendicular to the optical path.

The physical object 5 is irradiated with the light 8 having passed through the screen 21 or the light 8 reflected by the screen 21. The beam diameter f₃ of light 26 on a surface 5s of the physical object 5 is larger than both the beam diameter f₁ of light on the diffusing surface 20s and the beam diameter f₂ of light on the screen 21. The distance WD does not need to be that great in order for the physical object 5 to be illuminated at a wide angle. The light 8 enters the physical object 5. A portion of the light 8 turns into directly-reflected light (not illustrated) that is reflected by the surface 5s, and another portion of the light 8 turns into internally-scattered light 9.

The laser device 6 further includes an optical member 28 having a first surface 1 and a second surface that face each other. In the example shown in Fig. 1, the diffusing member 20 and the screen 21 are formed integrally with the optical member 28. The optical member 28 has translucency. The optical member 28 is for example a glass is a planar substrate of glass or resin. The diffusing member 20 is disposed on the first surface, and the screen 21 is disposed on the second surface. The optical member 28, on which the diffusing member 20 and the screen 21 are disposed, is placed so that the diffusing member 20 faces the laser light source 1. Placing the diffusing member 20 and the screen 21 on the optical member 28 so that the diffusing member 20 and the screen 21 face each other brings about a structure stabilization effect. Further, the optical member 28, the diffusing member 20, and the screen 21 may be used as a single optical component by being integrally molded by injection molding of resin. The integral molding is advantageous in terms of cost and positioning.

The inventors found the following. The light 31, which is diverging light, emitted from the laser light source 31 enters the diffusing member 20 and turns into the light 23, which is refracted light or diffracted light emitted spread from the diffusing member 20. The light 23 travels in a straight line in a particular direction without being multiply scattered. Therefore, there are no temporal variations in time distribution of optical power no matter where on the screen 21 a time distribution is observed. Further, a time distribution of optical power is the same in shape as a time distribution of the optical power of the light 31. That is, in a case where a short optical pulse of, for example, approximately 1 to 20 ns has been emitted from the laser light source 1, the falling time of an optical pulse of the laser light source 1 can be considered to be the same as the falling time of an optical pulse 25 on the screen 21.

The screen 21 may include, for example, a plurality of depressions and a plurality of projections alternately arranged two-dimensionally on the surface of the screen 21, and the depth of each of the plurality of depressions and the height of each of the plurality of projections may each range from 2 µm to 30 µm. Alternatively, the screen 21 may include a first layer and a second layer, and the refractive index of the first layer may be different from the refractive index of the second layer. For example, the second layer may be formed by applying paint to a surface of the first layer. Alternatively, the screen 21 may include a plurality of first parts and a plurality of second parts alternately arranged two-dimensionally on the surface of the screen 21, and the refractive index of each of the plurality of first parts may be different from the refractive index of each of the plurality of second parts. It is desirable that multiple scattering not occur on the screen 21. The illustrated structure is a structure in which multiple scattering hardly occurs. The inventors found that multiple scattering hardly occurs when such a screen 21 having a surface to which paint has been applied or a screen 21 varying in refractive index from position to position satisfies 1/µₛ ≤ d ≤ 1/µₛ', where d is the thickness of the member, µₛ' is the equivalent scattering coefficient of the member, and µₛ is the scattering coefficient of the member. In a case where the physical object 5 is irradiated with the light 8 having passed through such a screen 21, the falling time of an optical pulse on the surface 5s of the physical object 5 can be considered to be substantially the same as the falling time of an optical pulse of the laser light source 1, so that the influence of multiple scattering is small. Accordingly, the laser device 6 is applicable to a high S/N ratio time-resolved measurement.

Further, the apparent light source size is enlarged from f₁ to f₂. As a result, AEL and MPE for Class 1 can be increased by the size of the square of a scale of enlargement (f₂/f₁).

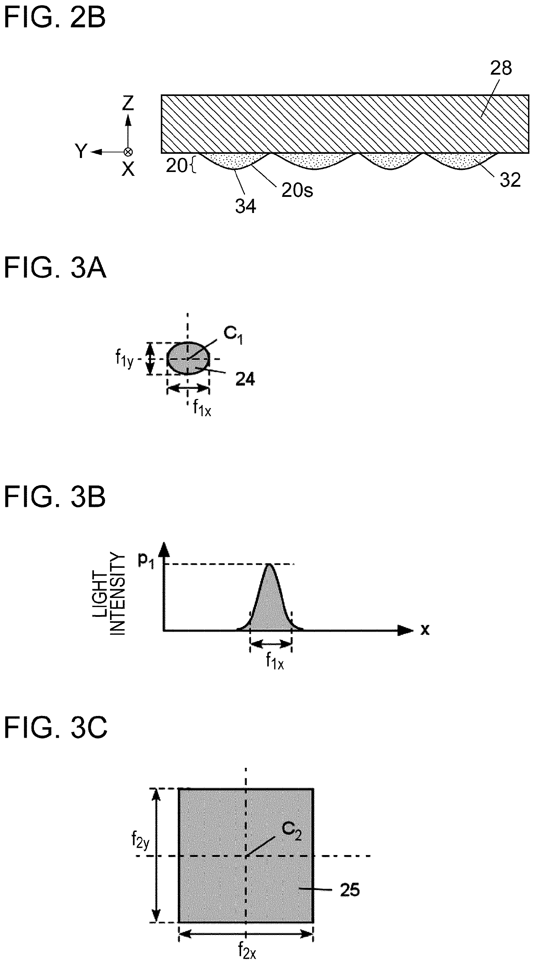

Figs. 2A and 2B are a plan view and a cross-sectional view, respectively, schematically showing a structure of the diffusing member 20 of the laser device 6 according to the present disclosure. The cross-section in the example shown in Fig. 2B is equivalent to the cross-section taken along IIB-IIB in the example shown in Fig. 2A.

The diffusing member 20 is placed on one of the two surfaces of the optical member 28. The diffusing member 20 has a function of further spreading the light 31, which is emitted light from the laser light source 1. As shown in Fig. 1, the diffusing member 20 converts the light 31 into the light 23, which is refracted light or diffracted light. The light 31 has a maximum angle of emergence θ. The light 23 emitted from the diffusing member 20 has a spread angle θ₁ that is larger than an angle sin⁻¹ (sinθ/n) of incidence on the optical member 28 with a refractive index n in the absence of the diffusing member 20. Therefore, θ₁ > sin⁻¹(sinθ/n) holds. In order to perform a time-resolved measurement with a reduced loss of light, it is desirable that the scattering member 20 not be a microparticle-containing scatterer.

In the laser device 6 embodiment, the diffusing member 20 includes a lens array 32 on the diffusing surface 20s. The lens array 32 diffuses light by refracting or diffracting it. The lens array 32 is formed, for example, by a transparent resin containing no microparticles. Although, in the examples shown in Figs. 2A and 2B, the lens array 32 is a 4 × 4 refractive lens array having four lenses arranged in both the X direction and the Y direction, the lens array 32 may alternatively be a diffractive lens array. The number of lenses may be set according to the specifications. The number of lenses may be increased by reducing the size of each lens. Further, although the lenses shown are convex in shape, they may alternatively be concave in shape. Concave lenses and convex lenses may be randomly arranged to form a lens array.

Random variations in the centers 34 of the lenses, in the film thickness distributions of the lenses, and in lens boundaries 35 in an XY plane in the lens array 32 bring about an effect of reducing diffraction noise in the light 8. In the example shown in Fig. 2A, the film thickness distributions are represented by contour lines 33 of certain heights.

The light 31 emitted from the laser light source 1 has a Gaussian distribution of light intensity. By passing through each convex lens in the lens array 32, the light 31 having a Gaussian distribution of light intensity turns into a plurality of rays from each separate convex lens that overlap one another to form a wholly flat distribution of light intensity.

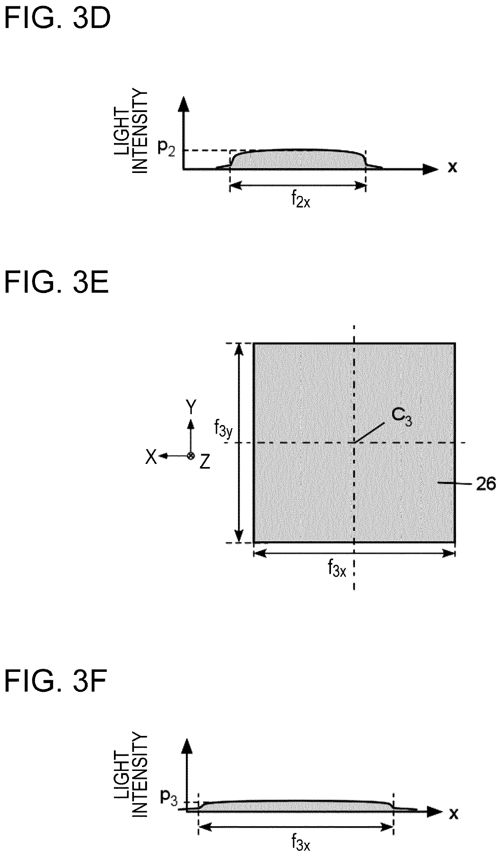

Fig. 3A is a diagram schematically showing a shape of light on the diffusing surface 20s of the laser device 6 according to the present disclosure, and Fig. 3B is a diagram schematically showing a light intensity distribution of the light on the diffusing surface 20s of the laser device 6. Fig. 3C is a diagram schematically showing a shape of light on the screen 21 of the laser device 6 according to the present disclosure, and Fig. 3D is a diagram schematically showing a light intensity distribution of the light on the screen 21 of the laser device 6. Fig. 3E is a diagram schematically showing a shape of light on the surface 5s of the physical object 5 according to the present disclosure, and Fig. 3F is a diagram schematically showing a light intensity distribution of the light on the surface 5s of the physical object 5.

In a case where a common semiconductor laser light source is used as the laser light source 1, the light 31, which is emitted from the laser light source 1, has a Gaussian distribution with different angles of emergence in the X direction and the Y direction. Accordingly, as shown in Fig. 3A, the shape of the light 24 on the diffusing member 20 is for example an elliptical shape having a long axis in the X direction. C₁ represents the center of the elliptical shape. As shown in Fig. 3B, the maximum light intensity of the light 24 is P₁. The beam diameters of 1/e² of the light 24 in the X direction and the Y direction are f₁ₓ and f_{1y}, respectively, and for example, when d₁ = 4 mm, f₁ₓ = 2 mm and f_{1y} = 1 mm. The apparent light source size in the diffusing member 20 is the average of the beam diameters of 1/e² in the X direction and the Y direction. The apparent light source size is 1.5 mm. The average of the beam diameters is herein sometimes referred to simply as "beam diameter".

Using the lens array 32 as the diffusing member 20 makes it possible to convert a Gaussian distribution of light intensity into a substantially flat distribution of light intensity on the screen 21. The beam diameters of 1/e² in the X direction and the Y direction are f₂ₓ and f_{2y}, which are substantially the same; for example, f₂ₓ = f_{2y} = 10 mm. As shown in Fig. 3C, the shape of the light 25 on the screen 21 can be made equal to the shape of a lens boundary 35 forming one lens. C₂ represents the center of the rectangular shape.

As shown in Fig. 3D, the maximum light intensity becomes P₂ and can be made much smaller than P₁. In terms of safety of the eyes, it is desirable that the maximum light intensity of the apparent light source be low. This is for the following reason. In a case where laser light has fallen on an eye, a speckle appears on the retina, as the laser light has coherence. This causes the light intensity to reach its maximum in certain places on the retina. If the maximum light intensity is too high, the retina might be damaged.

Accordingly, the diffusing member 20 refracts or diffract the light 31 emitted from the laser light source 1, thereby reducing the intensity of the central part of the light 31 and increases the intensity of the peripheral part of the light 31. The laser device 6 is required to have such characteristics as to be able to maximize total optical power while minimizing light intensities in positions on the screen 21, which is the apparent light source. A light intensity distribution of the light 25 on the screen 21 may be a flat distribution such as that shown in Fig. 3D.

For a reduction in size of the laser device 6 in the Z direction, it is preferable that d₁ + d₂ be small. Meanwhile, the beam diameter of the light 25 on the screen 21, which is the apparent light source size, may be not smaller than 10 mm. Further, d₁ may be reduced so that the beam diameter of the light 24 on the diffusing surface 20s is smaller than 10 mm. This is because, as mentioned above, when the apparent light source size is smaller than 10 mm, AEL and MPE for Class 1 can be increased in proportion to the square of the apparent light source size. For minimization of d₂, the beam diameter f of light on the screen 21 may be 10 mm.

A substantial spread angle θ₁-sin⁻¹(sinθ/n) that is obtained by the diffusing member 20 may be made larger than a substantial spread angle θ₂-sin⁻¹(nsinθ₁) that is obtained by the screen 21. This brings about an effect of making d₂ smaller. The substantial spread angle that is obtained by the diffusing member 20 means a measure of an angle by which oblique incident light is spread by the diffusing member 20. This substantial spread angle is equivalent to the difference between an angle of a ray of light in the absence of the diffusing member 20 and an angle of a ray of light in the presence of the diffusing member 20. The substantial spread angle that is obtained by the screen 21 means a measure of an angle by which oblique incident light is spread by the screen 21. This substantial spread angle is equivalent to the difference between an angle of a ray of light in the absence of the screen 21 and an angle of a ray of light in the presence of the screen 21.

In the laser device 6 the apparent light source size is enlarged, for example, from f₁ = 1.5 mm to f₂ = 10 mm. The scale of enlargement is f₂/f₁ = 6.7 times. Then, AEL and MPE for Class 1 can be increased by 44 times, which is the size of the square of the scale of enlargement.

The light 26 on the surface 5s of the physical object 5 is light obtained by spreading the light 25 on the screen 21. Therefore, as shown in Fig. 3E, the beam diameters of 1/e² in the X direction and the Y direction are f₃ₓ and f_{3y}, which are substantially the same; for example, f₃ₓ = f_{3y} = 60 mm. As shown in Fig. 3F, the maximum light intensity P₃ of the light 26 is smaller than P₂, so that the light 26 has a substantially flat light intensity distribution.

The distance d₂ from the diffusing member 20 to the screen 21 may be made longer than the distance d₁ from the surface of emergence of the laser light source 1 to the diffusing member 20. This makes it possible to increase the scale of enlargement of the apparent light source size while holding d₁ + d₂ constant.

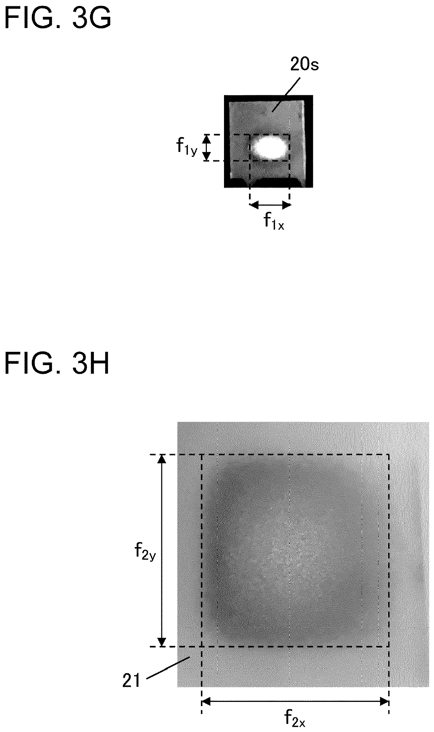

Fig. 3G is an example of a diagram showing a light intensity distribution on the diffusing surface 20s in a case where the diffusing member 20 having depressions and projections has been actually irradiated with red laser light, and Fig. 3H is an example of a diagram showing a light intensity distribution on the screen 21 in a case where the diffusing member 20 having depressions and projections has been actually irradiated with red laser light. In the example shown in Fig. 3G, a Gaussian distribution with a small beam diameter and a high light intensity is shown in the diffusing surface 20s. The color of white indicates a high light intensity. Meanwhile, in the example shown in Fig. 3H, a Gaussian distribution with a large beam diameter and a low light intensity is shown on the screen 21. The color of black indicates a low light intensity. As shown in Figs. 3G and 3H, it is found that light having passed through the diffusing member 20 exhibits a substantially flat distribution with a much-enlarged beam diameter and a low intensity distribution.

In the absence of the screen 21, light having a high light intensity distribution as shown in Fig. 3G is directly viewed by a subject as the apparent light source. This causes the subject to feel dazzled and have a feeling of discomfort. Further, in terms of safety of the eyes, it is not desirable to directly view light having a high light intensity distribution. Meanwhile, in the presence of the screen 21, a light having a low light intensity distribution as shown in Fig. 3H can be safely directly viewed by the subject as the apparent light source.

Next, a laser sensing apparatus according to Embodiment 1 of the present disclosure is described.

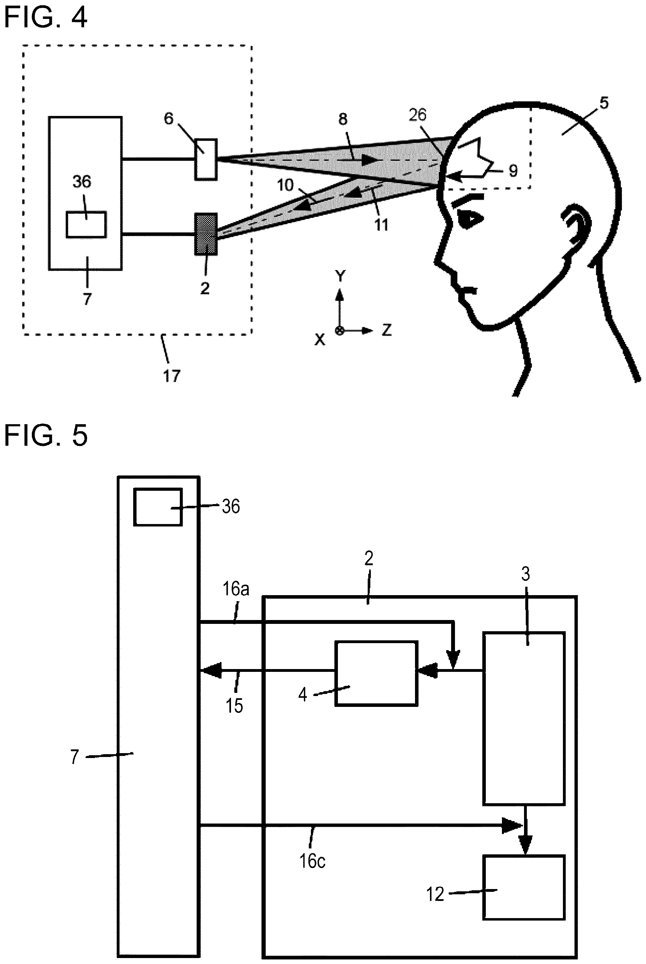

Fig. 4 is a schematic view for explaining a configuration of an optical sensing apparatus 17 according to Embodiment 1 of the present disclosure and how a biometric measurement is carried out. Fig. 5 is a diagram schematically showing an internal configuration of a control circuit 7 and a photodetector 2 of the optical sensing apparatus 17 according to Embodiment 1 of the present disclosure and the flow of signals.

The light sensing apparatus 17 according to Embodiment 1 includes the aforementioned laser device 6, the photodetector 2, and the control circuit 7.

The control circuit 7 controls the laser device 6 and the photodetector 2. The laser light source 1 can generate almost any optical pulse by starting and stopping the emission of light and changing light emission powers in accordance with instructions from the control circuit 7. Further, the control circuit 7 includes a signal processing circuit 36 that processes an electric signal 15 (hereinafter referred to simply as "signal") that is outputted from the photodetector 2. The electric signal 15 contains information on an internal state. The signal processing circuit 36 generates internal information on the physical object 5 by performing a computation involving the use of a plurality of signals outputted from the photodetector 2.

The control circuit 7 may be an integrated circuit having a processor such as a central processing unit (CPU) and a memory. For example, by executing a program stored in the memory, the control circuit 7 causes the laser device 6 to emit light and causes the photodetector 2 to detect the light in synchronization with the emission of the light by the laser device 6.

The photodetector 2 detects reflected scattered light 11 reflected and/or scattered by a physical object 5 located away from the laser device 6 and outputs an electric signal 15. The photodetector 2 includes a photoelectric converter 3 that generates signal charge corresponding to the amount of light received, a storage 4 in which signal charge is stored, and a drain 12 through which signal charge is discharged. The photoelectric converter 3 may include, for example, a photodiode. Signal charge produced by the photoelectric converter 3 is stored in the storage 4 or discharged through the drain 12. The timings of signal storage and discharge are controlled by the control circuit 7 and an internal circuit of the photodetector 2. The internal circuit of the photodetector 2 involved in this control is herein sometimes referred to as "electronic shutter".

In the present embodiment, the physical object 5 is the forehead of a person. Information on cerebral blood flow can be acquired by irradiating the forehead with light and detecting the resulting scattered light. The "scattered light" contains reflected scattered light and transmitted scattered light. In the following description, the reflected scattered light is sometimes simply referred to as "reflected light".

Present in the interior of the forehead, which is the physical object 5, are the scalp (approximately 3 to 6 mm thick), the skull (approximately 5 to 10 mm thick), the cerebrospinal fluid layer (approximately 2 mm thick), and the brain tissue, starting from the surface. The ranges of thicknesses in parentheses mean that there are differences between individuals. Blood vessels are present in the scalp and in the brain tissue. Accordingly, blood flow in the scalp is called "scalp blood flow", and blood flow in the brain tissue is called "cerebral blood flow". In a cerebral function measurement, a measurement object is a part where there is cerebral blood flow.

A living body is a scatterer. A portion of the light 8 emitted toward the physical object 5 returns as directly-reflected light 10 to the optical sensing apparatus 17. Another portion of the light enters the interior of the physical object 5 and gets diffused, and a portion of it is absorbed. The light having entered the interior of the physical object 5 turns into internally-scattered light 9 containing information on blood flow that is present in a range of depth of approximately 10 to 18 mm from the surface, i.e. cerebral blood flow. The internally-scattered light 9 returns to the optical sensing apparatus 17 as reflected scattered light 11 from the interior. The directly-reflected light 10 and the reflected scattered light 11 are detected by the photodetector 2.

The time from the emission of the directly-reflected light 10 from the laser device 6 to arrival of the directly-reflected light 10 at the photodetector 2 is relatively short, and the time from the emission of the reflected scattered light 11 from the interior from the laser device 6 to arrival of the reflected scattered light 11 from the interior at the photodetector 2 is relatively long. Among them, the component required to be detected at a high S/N ratio is the reflected scattered light 11 from the interior, which has the information on cerebral blood flow.

It should be noted that the transmitted scattered light, as well as the reflected scattered light, may be used in carrying out a biological measurement other than a cerebral blood flow measurement. In a case where information on blood other than cerebral blood flow is acquired, the part being tested may be a part other than the forehead (e.g. an arm, a leg, or the like). In the following description, unless otherwise noted, the physical object 5 is the forehead. The subject is a human, but may alternatively be a non-human animal having skin and having a hairless part. The term "subject" as used herein means specimens in general including such animals.

In order to quantify the light amounts of the directly-reflected light 10 and the reflected scattered light 11 that are detected by the photodetector 2, the inventors ran a simulation of an optical pulse response assuming, as the physical object 5, a phantom mimicking the head of a typical Japanese. Specifically, the inventors calculated through a Monte Carlo analysis a time distribution of optical power, i.e. an optical pulse response, that is detected by the photodetector 2 in a case where an optical pulse 8 is emitted toward a physical object 5 located at a distance of, for example, 15 cm from the laser device 6.

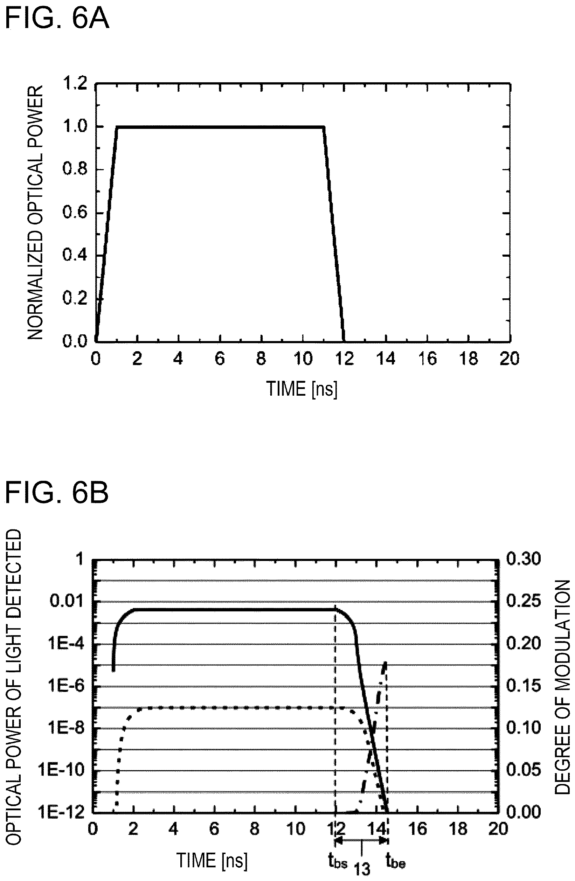

Fig. 6A is a diagram showing an example of a time distribution of a single optical pulse that is emitted light. In this example, the optical pulse has a wavelength λ of 850 nm and a full width at half maximum of 11 ns. This single optical pulse has a typical trapezoidal shape whose rising and falling times are each 1 ns. The term "rising time" as used herein means the time it takes for an optical power to increase from a peak value of 0% to 100%, and the period of time is referred to as "rising period". The term "falling time" means the time it takes for an optical power to decrease from a peak value (100%) to zero (0%), and the period of time is referred to as "falling period". The example shown in Fig. 6A assumes that the emission of the single optical pulse starts at a time t = 0 and completely stops at t = 12 ns.

Since the velocity of light c is 300000 km/s and the distance from the laser device 6 to the physical object 5 is 15 cm, the time t from the emission of the light 8, which is irradiating light, to the arrival of the light 8 at the surface of the physical object 5 is 0.5 ns. The time it takes for the light 8 to arrive at a surface of the photodetector 2 after being directly reflected by the surface of the physical object 5 and turning into the directly-reflected light 10 is 1 ns. Accordingly, the time it takes for the light to be detected on the photodetector 2 is 1 ns or longer.

The optical sensing apparatus 17 measures the amount of change in light amount of the reflected scattered light 11 from the interior of the physical object 5 on the basis of changes in oxyhemoglobin concentration and deoxyhemoglobin concentration in the cerebral blood flow. The brain tissue has an absorber whose absorption coefficient and scattering coefficient vary according to changes in cerebral blood flow. In a stationary state, it is possible to model the interior of the brain as uniform brain tissue and conduct a Monte Carlo analysis. The term "changes in blood flow" as used herein means temporal changes in blood flow.

Fig. 6B is a diagram showing total optical power (solid line) detected in a stationary state, an amount of change (dotted line) in optical power corresponding to an amount of change in cerebral blood flow, and a time distribution of degrees of modulation (dot-and-dash line). The term "degree of modulation" means a value obtained by dividing, by the total optical power detected in the stationary state, the amount of change in optical power corresponding to the amount of change in cerebral blood flow. In Fig. 6A, the vertical axis is expressed by a linear display, and in Fig. 6B, the vertical axis is expressed by a logarithmic display.

The amount of change in optical power corresponding to the amount of change in cerebral blood flow, which is included in the total optical power detected in the stationary state, is only approximately 2 × 10⁻⁵.

Let it be assumed that t_{bs} is the time at which the light power starts to decrease on the photodetector 2 and t_{be} is the time at which the light power completely decreases to a noise level. As shown in Fig. 6B, it is found that the proportion of signals that indicate changes in cerebral blood flow becomes higher in a falling period 13 of light during which the time t is not shorter than t_{bs} and not longer than t_{be}. As the second half of the falling period 13 of light passes, the light amount decreases and noise increases accordingly. However, the degree of modulation becomes higher. Of the light falling period 13 of light t_{bs} ≤ t ≤ t_{be}, the power of light at and after t = 13.5 ns, for example, is approximately 1/100 of the total optical power at which the light 8, which is an optical pulse, was detected. In a case where light arriving during the falling period 13 is subjected to a time-resolved measurement with use of the function of an electronic shutter of the photodetector 2, the proportion of optical power corresponding to the amount of change in cerebral blood flow increases to 7% of the total optical power that is detected at and after t = 13.5 ns. This makes it possible to sufficiently acquire signals that indicate changes in cerebral blood flow. Without use of the electronic shutter, the proportion of changes in cerebral blood flow is approximately 2 × 10⁻⁵.

Accordingly, signals that indicate changes in cerebral blood flow can be detected by using the photodetector 2 to receive a component of the reflected scattered light 11 included in the falling period 13 of an optical pulse from the physical object 5 and detect changes in optical power thereof.

The emission of an optical pulse and the detection of light in the light sensing apparatus 17 of to the present embodiment are described on the basis of the aforementioned principle of measurement of changes in cerebral blood flow.

In a case where the subject 5 is a human as in the case of the present embodiment, AEL and MPE for Class 1 need to be met for safety of the eyes. As described above, the laser device 6 of the present embodiment can increase AEL and MPE. However, the S/N ratio of signal light is not enough in many cases of cerebral function measurements. Accordingly, improvement in S/N ratio is usually brought about by repeatedly performing optical pulse emission and signal detection.

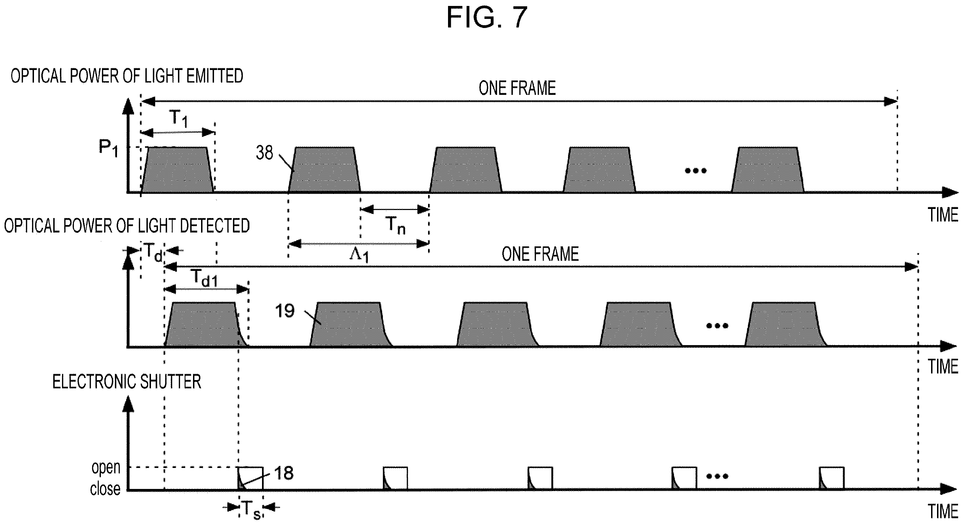

Fig. 7 is a diagram schematically showing a time distribution (upper row) of optical pulses 38 emitted from the laser device 6, a time distribution (middle row) of optical power detected by the photodetector 2 of the optical sensing apparatus 17, and timings and charge storage (lower row) of an electronic shutter according to the present disclosure.

In the optical sensing apparatus 17 the control circuit 7 causes the laser device 6 to irradiate the physical object 5 with at least one optical pulse 38. The control circuit 7 causes the photodetector 2 to detect a component of light included in a falling period of at least one reflected optical pulse 19 returning from the physical object 5 and output an electric signal 15 representing the amount of light detected.

As shown in the upper row of Fig. 7, the laser light source 1 emits optical pulses 38 in sequence, for example, in a cycle Λ₁. An optical pulse 38 has a pulse width T₁ and a maximum optical power P₁. Tₙ (= Λ₁ - T₁) represents a duration of time during which no optical pulse 38 is present.

As shown in the middle row of Fig. 7, a distribution of a reflected optical pulse 19 detected by the photodetector 2 in correspondence with an optical pulse 38 has a pulse shape whose lower slope is slightly spread. This is attributed to the occurrence of a time lag under the influence of internal scattering in the physical object 5. The pulse width T_{d1} is slightly wider than T₁.

The photodetector 2 photoelectrically converts, through the photoelectric converter 3 of the photodetector 2, a component of light in a reflected optical pulse 19 included in a falling period 13 and stores signal charge 18 in the storage 4.

In the present embodiment, the pulse width T₁ of an optical pulse 38 ranges from 11 to 22 ns. These optical pulses 38 may be repeatedly emitted, for example, approximately 1000 times to 1000000 times in a time cycle Λ₁ of approximately 55 ns to 110 ns. In this way, one frame is composed. Laying frames side by side can compose a moving image.

In the optical sensing apparatus 17 of the present embodiment, the photodetector 2 includes an electronic shutter that switches between storing signal charge and not storing signal change and the storage 4. The electronic shutter is a circuit that controls storage and discharge of signal charge generated by the photoelectric converter 3.

A component of light included in a falling period 13 of a reflected optical pulse 19 is photoelectrically converted by the photoelectric converter 3. After that, the storage 4 is selected (that is, the electronic shutter is kept open) in accordance with a control signal 16a from the control circuit 7, and signal charge 18 is stored for a period of time T_{S} of, for example, 11 to 22 ns. After passage of the period of time Ts, the drain 12 is selected (that is, the electronic shutter is kept close) in accordance with a control signal 16c from the control circuit, and charge from the photoelectric converter 3 is released.

Accordingly, a repetitive string of components of light included in falling periods 13 of reflected optical pulses 19 is stored in the storage 4 by photoelectric conversion as one frame of signal charge 18 in correspondence with a repetitive pulse string of optical pulses 38. After the end of one frame, the signal charge 18 is outputted to the control circuit 7 as an electric signal 15. The electric signal 15 contains information on cerebral blood flow.

After the emission of optical pulses 38, ambient noise may be measured by keeping the electronic shutter open and closed for the same length of time and the same number of times in the absence of light emission. The S/N ratios of the signals can be improved by subtracting the value of the ambient noise from each of the signal values.

The configuration of the photodetector 2 shown in Fig. 5 is equivalent to one pixel. This makes it possible to acquire biological information about averaged blood flow within the physical object 5.

Alternatively, as the photodetector 2, an image sensor including, for each pixel, a photoelectric converter 3, a storage 4, and an electronic shutter that switches between storing signal charge and not storing signal charge in the storage 4 may be used. In this case, the photodetector 2 is an image sensor having a plurality of photodetection cells arrayed two-dimensionally. Each of the photodetection cells stores, as signal charge 18, a component of light included in a falling period of a reflected optical pulse 19. Furthermore, each of the photodetection cells outputs an electric signal 15 representing the total amount of signal charge stored. This makes it possible to acquire biological information about the blood flow of the physical object 5 as a moving image including a plurality of frames.

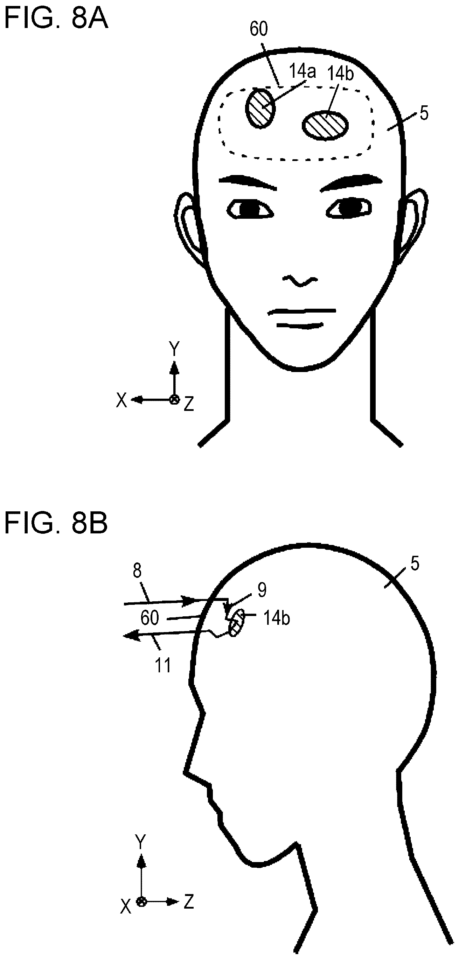

Next, the superimposition of the information on cerebral blood flow onto the electric signal 15 is described with reference to Figs. 8A and 8B.

Fig. 8A is a front view showing changes in blood flow that are present inside the physical object 5. Fig. 8B is a side cross-sectional view showing changes in blood flow that are present in the physical object 5. In the examples shown in Figs. 8A and 8B, regions 14a and 14b are shown that indicate internal blood flow at approximately 10 to 18 mm from the surface. The internal blood flow here is cerebral blood flow. Attention is paid to the optical path through which the light 8, which is irradiating light, enters the physical object 5 and is detected as the internally-scattered light 9 from the interior by the photodetector 2. The internally-scattered light 9, albeit depending on a blood flow distribution, passes through the regions 14a and 14b. Furthermore, the internally-scattered light 9 is repeatedly scattered or absorbed and comes out of the physical object 5 as the reflected scattered light 11 from the interior.

Next, a method for acquiring a distribution that indicates changes in blood flow in the physical object 5 is described.

First, the control circuit 7 causes the photodetector 2, which is an image sensor, to output the following first and second image signals. The first image signal represents a two-dimensional distribution of the total amount of signal charge 18 stored in the plurality of photodetection cells during a first period. The second image signal represents a two-dimensional distribution of the total amount of signal charge 18 stored in the plurality of photodetection cells during a second period preceding the first period.

Next, the signal processing circuit 36 receives the first and second image signals from the photodetector 2. After that, the signal processing circuit 36 generates a difference image representing the difference between an image represented by the first image signal and an image represented by the second image signal.

The difference image is equivalent to a distribution that indicates changes in cerebral blood flow in a part being tested 60. It is assumed herein that the difference image is an image that uses the second image signal as a reference value and displays an increase or decrease in the first image signal from the reference value. When the signal processing circuit 36 receives the second image signal only once and repeatedly receives the first image signal every one-frame cycle, a moving image representing a distribution that indicates changes in blood flow in the physical object 5 is obtained.

Next, a method for improving the size of a cerebral blood flow region detected to an actual size is described.

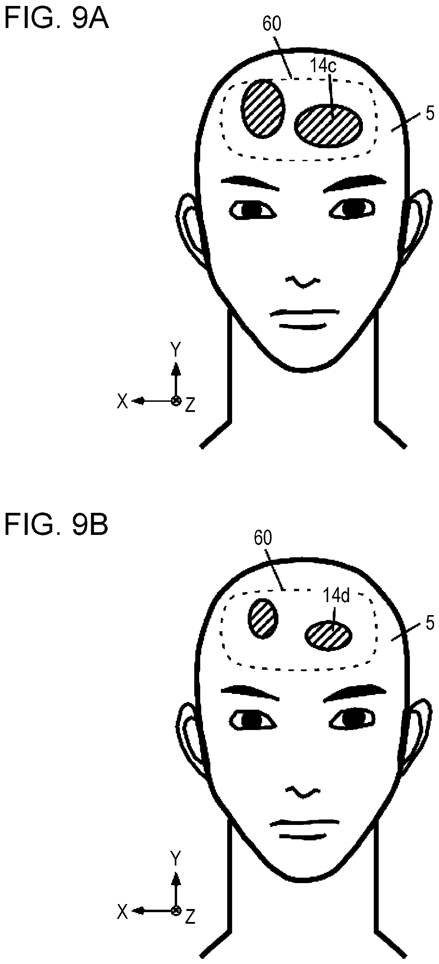

Fig. 9A is a diagram schematically showing changes in blood flow in the interior of the physical object as detected by irradiation with an optical pulse. Fig. 9B is a diagram schematically showing changes in blood flow in the interior of the physical object 5 as image-corrected by image computations.

The signal processing circuit 36 generates blood flow information on the interior of the physical object 5 through the use of an electric signal 15 representing an amount of signal charge 18. The electric signal 15 contains the blood flow information on the interior of the physical object 5.

In the example shown in Fig. 9A, the two-dimensional image represents a distribution of a region 14c of change in cerebral blood flow. The region 14c of change in cerebral blood flow is in a spread state due to scattering of cerebral blood flow in the interior. To address this problem, the signal processing circuit 36 makes an image correction by guessing the scattering state through a diffusion equation or a Monte Carlo analysis. By so doing, the signal processing circuit 36 generates an actual-size two-dimensional image representing a distribution of a region 14d of change in cerebral blood flow such as that shown in Fig. 9B. This two-dimensional image is a desired image that indicates changes in cerebral blood flow.

Next, laser devices 6 according to modifications of Embodiment 1 of the present disclosure are described.

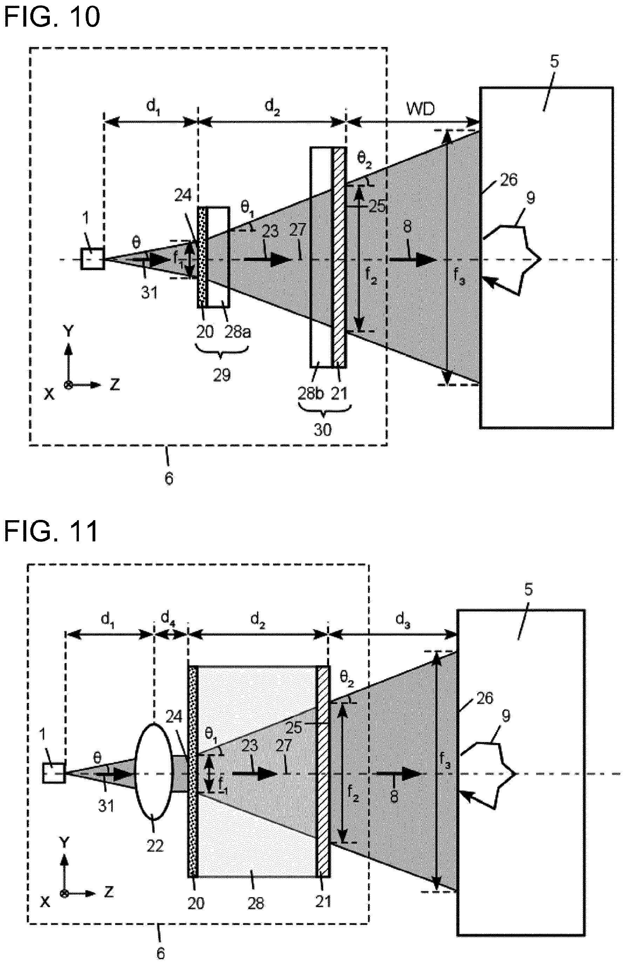

Fig. 10 is a schematic view for explaining a configuration of a laser device 6 according to the present disclosure and how a physical object 5 is irradiated with light 8 emitted from the laser device 6.

Instead of the optical member 28, the laser device 6 further includes an optical member 28a having two surfaces opposite to each other and an optical member 28b having two surfaces opposite to each other. In the laser device 6, the diffusing member 20 and the screen 21 are not disposed on an identical optical member but disposed on the separate optical members 28a and 28b, respectively. The diffusing member 20 is on one of the two surfaces of the optical member 28a, and the screen 21 is on one of the two surfaces of the optical member 28b. The other of the two surfaces of the optical member 28a faces the other of the two surfaces of the optical member 28b. The phrase "two surfaces opposite to each other" as used herein encompasses a case where the two surfaces are not parallel to each other. In this example, the optical members 28a and 28b have translucency. The optical members 28a and 28b are for example planar substrates of glass or resin.

The diffusing member 20 and the optical member 28a are combined to serve as a diffusing structure 29, and the screen 21 and the optical member 28b are combined to serve as a screen structure 30. The diffusing structure 29 can be fabricated by forming, on a larger optical member 28a, a diffusing member 20 of the same size as the optical member 28a and cutting out a desired size. The diffusing structure 29 is much smaller in size than the screen structure 30. Accordingly, the cost of the diffusing structure 29 can be reduced.

Further, since the diffusing member 20 is on that one of the two surfaces of the optical member 28a which faces the laser light source 1, it is possible to make the scale of enlargement of the apparent light source larger than it is when the diffusing member 20 is on the opposite surface. Further, since the screen 21 is on that one of the two surfaces of the optical member 28b which is opposite to the surface facing the laser light source 1, it is possible to make the scale of enlargement of the apparent light source larger than it is when the screen 21 is on the surface facing the laser light source 1.

Fig. 11 is a schematic view for explaining a configuration of a laser device 6 according to the present disclosure and how a physical object 5 is irradiated with light 8 emitted from the laser device 6.

The laser device 6 further includes a collimator lens 22 located between the laser light source 1 and the diffusing member 20. In the laser device 6, the emitted light 31 from the laser light source 1 enters the diffusing member 20 after being converted by the collimator lens 22 into parallel light. The diffusing member 20 is designed in accordance with parallel light. Accordingly, it is not necessary to design the diffusing member 20 in accordance with diverging light serving as the light 31 emitted from the laser light source 1, so that the design of the diffusing member 20 is simplified.

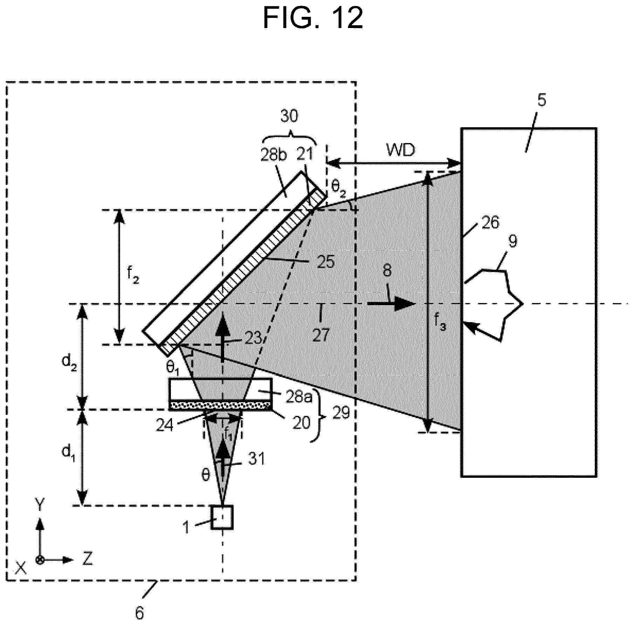

Fig. 12 is a schematic view for explaining a configuration of a laser device 6 according to the present disclosure and how a physical object 5 is irradiated with light 8 emitted from the laser device 6.

In the laser device 6 the screen structure 30 including the screen 21 is placed at a slant of, for example, 45 degrees with respect to the optical path of the light 23, which is refracted light or diffracted light emitted from the diffusing member 20. The diffusing member 20 is on one of the two surfaces of the optical member 28a, and the screen 21 is on one of the two surfaces of the optical member 28b. The other of the two surfaces of the optical member 28a faces the surface of the screen 21. The other of the two surfaces of the optical member 28a is tilted with respect to the surface of the screen 21. In this example, the optical member 28a has translucency, and the optical member 28b has reflectivity. The optical member 28a is for example a planar substrate of glass or resin. The optical member 28b is for example a planar substrate of metal. A usable example of the metal is aluminum.

The light 31 emitted in the Y direction is reflected by the screen 21 to be bent in the Z direction. The physical object 5 is irradiated with light bent in the Z direction. Such a folding optical system makes it possible to reduce the Z-direction size of the laser device 6.

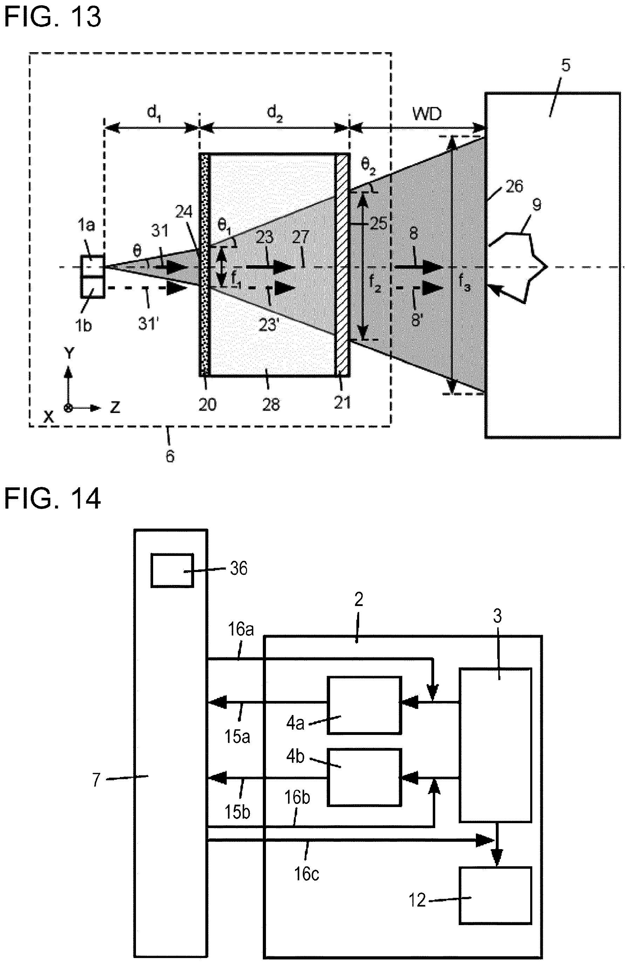

Next, an optical sensing apparatus according to an embodiment of the present disclosure is described with reference to Figs. 13 to 15.

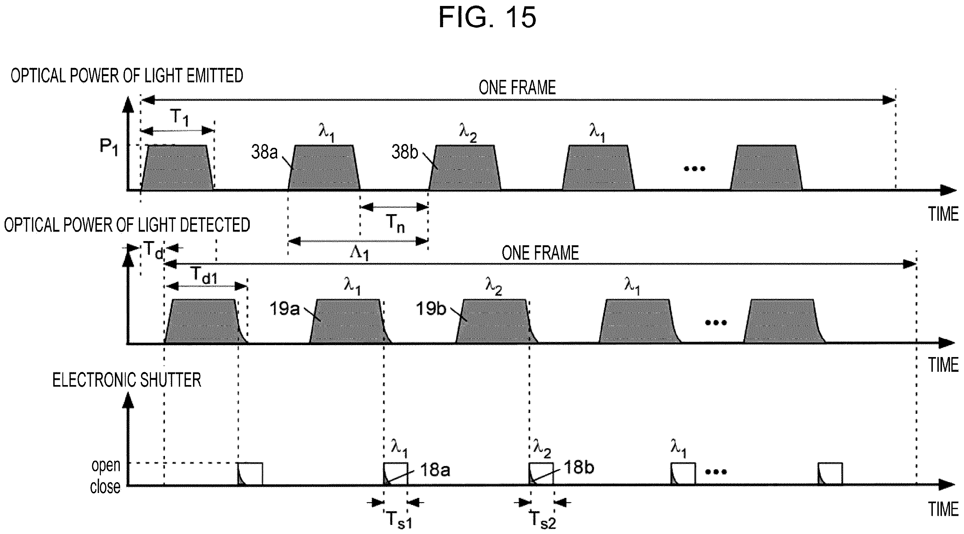

Fig. 13 is a schematic view for explaining a configuration of a laser device 6 according to an embodiment of the present disclosure and how a physical object 5 is irradiated with light 8 emitted from the laser device 6. Fig. 14 is a diagram schematically showing an internal configuration of a control circuit 7 and a photodetector 2 of an optical sensing apparatus 17 according to an embodiment of the present disclosure and the flow of signals. Fig. 15 is a diagram schematically showing a time distribution (upper row) of optical pulses emitted from the laser device 6, a time distribution (middle row) of optical power detected by the photodetector 2 of the optical sensing apparatus 17, and timings and charge storage (lower row) of an electronic shutter according to an embodiment of the present disclosure.

The laser device 6 comprises laser light source 1 which is a multiwavelength light source that emits at least two wavelengths of light and emits optical pulses in sequence for each separate wavelength. The optical sensing apparatus 17 includes the laser device 6 which has a multiwavelength light source.

The laser light source 1 includes, for example, a plurality of light-emitting elements 1a and 1b arranged side by side in the Y direction. The light-emitting element 1a emits light of a first wavelength range, and the light-emitting element 1b emits light of a second wavelength range that is different from the first wavelength range. The light-emitting elements 1a and 1b are for example laser chips.

The absorptance of oxyhemoglobin and deoxyhemoglobin varies, for example, at wavelengths of λ₁ = 750 nm and λ₂ = 850 nm. Therefore, computing two electric signals respectively obtained by using these two wavelengths makes it possible to measure the proportions of oxyhemoglobin and deoxyhemoglobin in the physical object 5.

When the physical object 5 is a forehead area of the head of a living body, the amount of change in cerebral blood flow in the frontal lobe, the amounts of change in oxyhemoglobin concentration and deoxyhemoglobin concentration, or the like can be measured. This makes sensing of information such as emotions possible. For example, in a centered state, there occur an increase in cerebral blood flow volume, an increase in amount of oxyhemoglobin, and the like.

Various combinations of wavelengths are possible. At a wavelength of 805 nm, the rates of absorption of oxyhemoglobin and deoxyhemoglobin become equal. Accordingly, in view of the biological window, for example, a wavelength of not shorter than 650 nm and shorter than 805 nm and a wavelength of longer than 805 nm and not longer than 950 nm may be combined. Furthermore, a third wavelength of 805 nm may be used in addition to the two wavelengths. In a case where three wavelengths of light are used, three laser chips are needed; however, since information on the third wavelength is obtained, utilizing the information may make computations easy.

The photodetector 2 of the optical sensing apparatus 17 of the present embodiment includes an electronic shutter that switches between storing signal charge and not storing signal charge and two storages 4a and 4b. An optical pulse 38a of a wavelength λ₁ is emitted from the light-emitting element 1a. The photoelectric converter 3 photoelectrically converts a component of reflected scattered light 11 included in a falling period 13 of a reflected optical pulse 19a returning to the photodetector 2 in correspondence with the optical pulse 38a. After that, the storage 4a is selected in accordance with control signals 16a, 16b, and 16c from the control circuit 7, and a first signal charge 18a is stored for a period of time T_{S1} of, for example, 11 to 22 ns. After passage of the period of time T_{S1}, the drain 12 is selected in accordance with the control signals 16a, 16b, and 16c from the control circuit 7, and, and charge from the photoelectric converter 3 is released.

After this, the light-emitting element 1a is replaced by the light-emitting element 1b, which similarly emits an optical pulse 38b of a wavelength λ₂. The photoelectric converter 3 photoelectrically converts a component of reflected scattered light 11 included in a falling period 13 of a reflected optical pulse 19b returning to the photodetector 2 in correspondence with the optical pulse 38b. After that, the storage 4b is selected in accordance with the control signals 16a, 16b, and 16c from the control circuit 7, and a second signal charge 18b is stored for a period of time T_{S2} of, for example, 11 to 22 ns. After passage of the period of time T_{S2}, the drain 12 is selected in accordance with the control signals 16a, 16b, and 16c from the control circuit 7, and, and charge from the photoelectric converter 3 is released. These optical pulses 38a and 38b are repeatedly emitted in sequence and stored as one frame of first signal charge 38a and one frame of second signal charge 18b, respectively. After the end of one frame, the first signal charge 18a and the second signal charge 18b are outputted to the control circuit 7 as a first electric signal 15a and a second electric signal 15b, respectively.

From two pieces of image information acquired, two images representing two-dimensional concentration distributions of oxyhemoglobin and deoxyhemoglobin, respectively, can be generated as images that indicate changes in cerebral blood flow.

The present embodiment, too, makes it possible to provide a laser device 6 that emits light that increases MPE and AEL for Class 1 and can be applied to a time-resolved measurement and an optical sensing apparatus 17 including the same that can improve an S/N ratio.

The optical sensing apparatuses 17 have been described with reference to a case where information on the physical object 5 is cerebral blood flow. The information on the physical object 5 may be information on the distance from the physical object to the photodetector. In this case, the electric signal 15 contains a signal representing the distance. The optical sensing apparatuses 17 may be configured as TOF (time-of-flight) cameras that can increase irradiating power while ensuring safety.

In the foregoing, the laser devices 6 and the optical sensing apparatuses 17 including the same have been described. However, the present disclosure is not limited to these.

The laser devices, the optical sensing apparatuses including the laser devices, and a laser device based on a combination of the configurations of the optical sensing apparatuses are also encompassed in the present disclosure and can bring about similar effects.

### Industrial Applicability

The present disclosure is applicable to uses in such areas as non-contact measurements of living bodies or food, e.g. biomedical sensing or freshness sensing.

### Reference Signs List

1 Laser light source
2 Photodetector
3 Photoelectric converter
4 Storage
5 Physical object
5s Surface
6 Laser device
7 Control circuit
8, 23, 24, 25, 26, 31 Light
9 Internally-scattered light
10 Directly-reflected light
11 Reflected scattered light
12 Drain
13 Falling period
14a, 14b, 14c, 14d Region
15 Electric signal
16a, 16b, 16c Control signal
17 Optical sensing apparatus
18 Signal charge
19 Reflected optical pulse
20 Diffusing member
20s Diffusing surface
21 Screen
22 Collimator lens
27 Optical path
28, 28a, 28b Optical member
29 Diffusing structure
30 Screen structure
32 Lens array
33 Contour line
34 Center
35 Lens boundary
36 Signal processing circuit

## Claims

1. An optical sensing apparatus (17) comprising:
a laser device (6) including
a light source (1) that emits laser light (31),
a diffusing member (20) having a diffusing surface (20s) that crosses an optical path of the laser light, the diffusing member refracting or diffracting the laser light to make the laser light lower in intensity in a first portion including a center of a cross-section of the laser light that crosses the optical path, make the laser light higher in intensity in a second portion of the laser light that surrounds the first portion in the cross-section, and enlarge a beam diameter of the laser light, and
a screen (21) that crosses an optical path (27) of the laser light (23) having passed through the diffusing member such that a spot of light having passed through the screen or having been reflected by the screen appears on a surface of the screen, the beam diameter (f2) of the spot forming an apparent light source size of the laser device,
the diffusing member being configured such that the laser light spreads at a first substantial spread angle when the laser light passes through the diffusing member, the screen being configured such that the laser light spreads at a second substantial spread angle when the laser light passes through the screen or is reflected by the screen, and the first substantial spread angle being larger than the second substantial spread angle
the laser device being configured to irradiate a physical object (5) with the laser light (8) having passed through the screen or the laser light having been reflected by the screen
a photodetector (2) that is configured to detect at least a portion of the laser light (11) returning from the physical object and output an electric signal (15); and
a control circuit (7) that is configured to control the laser device and the photodetector,
wherein the control circuit causes the laser device to irradiate the physical object with at least one optical pulse (38) of the laser light and causes the photodetector to perform a time-resolved measurement of at least one reflected optical pulse (19) of the laser light returning from the physical object.

2. The optical sensing apparatus according to Claim 1, wherein a beam diameter of the laser light on the diffusing surface is smaller than 10 mm.

3. The optical sensing apparatus according to Claim 1 or 2, wherein a beam diameter of the laser light on the screen is not smaller than 10 mm.

4. The optical sensing apparatus according to any of Claims 1 to 3, wherein a beam diameter of the laser light on a surface of the physical object is larger than a beam diameter of the laser light on the screen.

5. The optical sensing apparatus according to any of Claims 1 to 4, wherein the diffusing member further includes a lens array on the diffusing surface.

6. The optical sensing apparatus according to any of Claims 1 to 5, wherein the screen includes a plurality of depressions and a plurality of projections alternately arranged two-dimensionally on a surface of the screen, and
a depth of each of the plurality of depressions and a height of each of the plurality of projections each range from 2 µm to 30 µm.

7. The optical sensing apparatus according to any of Claims 1 to 5, wherein the screen includes a first layer and a second layer, and
a refractive index of the first layer is different from a refractive index of the second layer.

8. The optical sensing apparatus according to any of Claims 1 to 5, wherein the screen includes a plurality of first parts and a plurality of second parts alternately arranged two-dimensionally on a surface of the screen, and
a refractive index of each of the plurality of first parts is different from a refractive index of each of the plurality of second parts.

9. The optical sensing apparatus according to any of Claims 1 to 8, wherein 1/µs ≤ d ≤ 1/µs', where d is the thickness of the screen, µs' is the equivalent scattering coefficient of the screen, and µs is the scattering coefficient of the screen.

10. The optical sensing apparatus according to any of Claims 1 to 9, wherein a distance from the diffusing member to the screen is longer than a distance from the light source to the diffusing member.

11. The optical sensing apparatus according to any of Claims 1 to 10, further comprising an optical member, disposed between the diffusing member and the screen, that has a first surface and a second surface opposite to the first surface,
wherein the diffusing member is disposed on the first surface, and
the screen is disposed on the second surface.

12. The optical sensing apparatus according to any of Claims 1 to 10, further comprising:
a first optical member having a first surface and a second surface opposite to the first surface; and
a second optical member having a third surface and a fourth surface opposite to the third surface,
wherein the first optical member and the second optical member are both disposed between the diffusing member and the screen,
the diffusing member is disposed on the first surface,
the screen is disposed on the fourth surface, and
the second surface faces the third surface.

13. The optical sensing apparatus according to any of Claims 1 to 10, further comprising:
a first optical member having a first surface and a second surface opposite to the first surface; and
a second optical member having a third surface and a fourth surface opposite to the third surface,
wherein the first optical member is disposed between the diffusing member and the screen,
the diffusing member is disposed on the first surface,
the screen is disposed on the third surface,
the second surface faces a surface of the screen,
the second surface is tilted with respect to the surface of the screen, and
the laser device irradiates the physical object with the laser light reflected by the surface of the screen.

14. The optical sensing apparatus according to any of Claims 1 to 13, further comprising a collimator lens located between the light source and the diffusing member.

15. The optical sensing apparatus according to any of Claims 1 to 14, wherein the control circuit causes the photodetector to detect a component of light included in a falling period of the at least one reflected optical pulse and output an electric signal representing an amount of the component, and
the falling period is a period from beginning to end of a decrease in intensity of the at least one reflected optical pulse.

16. The optical sensing apparatus according to Claim 15, wherein the electric signal contains information on an internal state of the physical object.

17. The optical sensing apparatus according to Claim 16, wherein the physical object is a living body, and
the electric signal contains information on blood flow in the living body.

18. The optical sensing apparatus according to Claim 16, wherein the physical object is a human forehead, and
the electric signal contains information on cerebral blood flow.

19. The optical sensing apparatus according to any of Claims 1 to 18, wherein the electric signal contains information on a distance from the physical object to the photodetector.

20. The optical sensing apparatus according to Claim 19, wherein the photodetector is a time-of-flight camera.

## Patentansprüche

1. Optische Erfassungsvorrichtung (17), die umfasst:
eine Laser-Einrichtung (6), die enthält:
eine Lichtquelle (1), die Laserlicht (31) emittiert,
ein streuendes Element (20) mit einer streuenden Fläche (20s), die einen Lichtweg des Laserlichtes kreuzt, wobei das streuende Element das Laserlicht bricht oder beugt, um Intensität des Laserlichtes in einem ersten Abschnitt zu verringern, der eine Mitte eines Querschnitts des Laserlichtes einschließt, der den Lichtweg kreuzt, Intensität des Laserlichtes in einem zweiten Abschnitt des Laserlichtes zu verstärken, der den ersten Abschnitt in dem Querschnitt umgibt, und einen Strahldurchmesser des Laserlichtes zu vergrößern, sowie
einen Schirm (21), der einen Lichtweg (27) des Laserlichtes (23) kreuzt, das das streuende Element durchlaufen hat, so dass ein Lichtfleck, der den Schirm durchlaufen hat oder von dem Schirm reflektiert worden ist, auf einer Oberfläche des Schirms erscheint, wobei der Strahldurchmesser (f2) des Flecks eine scheinbare Lichtquellengröße der Laser-Einrichtung bildet,
wobei das streuende Element so ausgeführt ist, dass sich das Laserlicht in einem ersten wesentlichen Ausbreitungswinkel ausbreitet, wenn das Laserlicht das streuende Element durchläuft, der Schirm so ausgeführt ist, dass sich das Laserlicht in einem zweiten wesentlichen Ausbreitungswinkel ausbreitet, wenn das Laserlicht den Schirm durchläuft oder von dem Schirm reflektiert wird, und der erste wesentliche Ausbreitungswinkel größer ist als der zweite wesentliche Ausbreitungswinkel,
die Laser-Einrichtung so ausgeführt ist, dass sie ein physisches Objekt (5) mit dem Laserlicht (8), das den Schirm durchlaufen hat, oder dem Laserlicht bestrahlt, das von dem Schirm reflektiert worden ist,
einen Photodetektor (2), der so ausgeführt ist, dass er wenigstens einen Teil des von dem physischen Objekt zurückkehrenden Laserlichtes (11) erfasst und ein elektrisches Signal (15) ausgibt; sowie
eine Steuerungs-Schaltung (7), die zum Steuern der Laser-Einrichtung und des Photodetektors ausgeführt ist,
wobei die Steuerungs-Schaltung die Laser-Einrichtung veranlasst, das physikalische Objekt mit wenigstens einem optischen Impuls (38) des Laserlichtes zu bestrahlen, und den Photodetektor veranlasst, eine zeit-aufgelöste Messung wenigstens eines reflektierten optischen Impulses (19) des von dem physikalischen Objekt zurückkehrenden Laserlichtes durchzuführen.

2. Optische Erfassungsvorrichtung nach Anspruch 1, wobei ein Strahldurchmesser des Laserlichtes auf der streuenden Fläche kleiner ist als 10 mm.

3. Optische Erfassungsvorrichtung nach Anspruch 1 oder 2, wobei der Strahldurchmesser des Laserlichtes auf dem Schirm nicht kleiner ist als 10 mm.

4. Optische Erfassungsvorrichtung nach einem der Ansprüche 1 bis 3, wobei ein Strahldurchmesser des Laserlichtes auf einer Oberfläche des physischen Objektes größer ist als ein Strahldurchmesser des Laserlichtes auf dem Schirm.

5. Optische Erfassungsvorrichtung nach einem der Ansprüche 1 bis 4, wobei das streuende Element des Weiteren eine Linsenanordnung auf der streuenden Fläche einschließt.

6. Optische Erfassungsvorrichtung nach einem der Ansprüche 1 bis 5, wobei der Schirm eine Vielzahl von Vertiefungen und eine Vielzahl von Vorsprüngen enthält, die abwechselnd zweidimensional auf einer Oberfläche des Schirms angeordnet sind, und
eine Tiefe jeder der Vielzahl von Vertiefungen und eine Höhe jedes der Vielzahl von Vorsprüngen jeweils von 2 µm bis 30 µm reichen.

7. Optische Erfassungsvorrichtung nach einem der Ansprüche 1 bis 5, wobei der Schirm eine erste Schicht und eine zweite Schicht enthält, und
ein Brechungsindex der ersten Schicht sich von einem Brechungsindex der zweiten Schicht unterscheidet.

8. Optische Erfassungsvorrichtung nach einem der Ansprüche 1 bis 5, wobei der Schirm eine Vielzahl erster Teile und eine Vielzahl zweiter Teile enthält, die abwechselnd zweidimensional auf einer Oberfläche des Schirms angeordnet sind, und
ein Brechungsindex jedes der Vielzahl erster Teile sich von einem Brechungsindex jedes der Vielzahl zweiter Teile unterscheidet.

9. Optische Erfassungsvorrichtung nach einem der Ansprüche 1 bis 8, wobei 1/µs ≤ d ≤ 1/µs' gilt und dabei d die Dicke des Schirms ist, µs' der äquivalente Streuungskoeffizient des Schirms ist und µs der Streuungskoeffizient des Schirms ist.

10. Optische Erfassungsvorrichtung nach einem der Ansprüche 1 bis 9, wobei ein Abstand von dem streuenden Element zu dem Schirm größer ist als ein Abstand von der Lichtquelle zu dem streuenden Element.

11. Optische Erfassungsvorrichtung nach einem der Ansprüche 1 bis 10, die des Weiteren ein optisches Element umfasst, das zwischen dem streuenden Element und dem Schirm angeordnet ist und das eine erste Fläche sowie eine der ersten Fläche gegenüberliegende zweite Fläche hat,
wobei das streuende Element an der ersten Fläche angeordnet ist, und
der Schirm an der zweiten Fläche angeordnet ist.

12. Optische Erfassungsvorrichtung nach einem der Ansprüche 1 bis 10, die des Weiteren umfasst:
ein erstes optisches Element, das eine erste Fläche sowie eine der ersten Fläche gegenüberliegende zweite Fläche aufweist; und
ein zweites optisches Element, das eine dritte Fläche sowie eine der dritten Fläche gegenüberliegende vierte Fläche aufweist,
wobei das erste optische Element und das zweite optische Element beide zwischen dem streuenden Element und dem Schirm angeordnet sind,
das streuende Element an der ersten Fläche angeordnet ist,
der Schirm an der vierten Fläche angeordnet ist, und
die zweite Fläche der dritten Fläche zugewandt ist.

13. Optische Erfassungsvorrichtung nach einem der Ansprüche 1 bis 10, die des Weiteren umfasst:
ein erstes optisches Element, das eine erste Fläche sowie eine der ersten Fläche gegenüberliegende zweite Fläche aufweist; und
ein zweites optisches Element, das eine dritte Fläche sowie eine der dritten Fläche gegenüberliegende vierte Fläche aufweist,
wobei das erste optische Element zwischen dem streuenden Element und dem Schirm angeordnet ist,
das streuende Element an der ersten Fläche angeordnet ist,
der Schirm an der dritten Fläche angeordnet ist,
die zweite Fläche einer Oberfläche des Schirms zugewandt ist,
die zweite Fläche in Bezug auf die Oberfläche des Schirms geneigt ist, und
die Laser-Einrichtung das physische Objekt mit dem von der Oberfläche des Schirms reflektierten Laserlicht bestrahlt.

14. Optische Erfassungsvorrichtung nach einem der Ansprüche 1 bis 13, die des Weiteren eine Kollimatorlinse umfasst, die zwischen der Lichtquelle und dem streuenden Element angeordnet ist.

15. Optische Erfassungsvorrichtung nach einem der Ansprüche 1 bis 14, wobei die Steuerungs-Schaltung den Photodetektor veranlasst, eine Komponente von Licht zu erfassen, die in einer abfallenden Periode des wenigstens einen reflektierten optischen Impulses eingeschlossen ist, und ein elektrisches Signal auszugeben, das einen Betrag der Komponente repräsentiert, und
die abfallende Periode eine Periode von Beginn bis Ende einer Abnahme von Intensität des wenigstens einen reflektierten optischen Impulses ist.

16. Optische Erfassungsvorrichtung nach Anspruch 15, wobei das elektrische Signal Informationen über einen inneren Zustand des physischen Objektes enthält.

17. Optische Erfassungsvorrichtung nach Anspruch 16, wobei das physische Objekt ein lebender Körper ist, und
das elektrische Signal Informationen über Blutstrom in dem lebenden Körper enthält.

18. Optische Erfassungsvorrichtung nach Anspruch 16, wobei das physische Objekt eine menschliche Stirn ist, und
das elektrische Signal Informationen über zerebralen Blutstrom enthält.

19. Optische Erfassungsvorrichtung nach einem der Ansprüche 1 bis 18, wobei das elektrische Signal Informationen über einen Abstand von dem physischen Objekt zu dem Photodetektor enthält.

20. Optische Erfassungsvorrichtung nach Anspruch 19, wobei der Photodetektor eine Laufzeit-Kamera ist.

## Revendications

1. Appareil de détection optique (17) comprenant :
un dispositif laser (6) qui comprend
une source lumineuse (1) qui émet une lumière laser (31),
un élément de diffusion (20) ayant une surface de diffusion (20s) qui croise un chemin optique de la lumière laser, l'élément de diffusion réfractant ou diffractant la lumière laser pour rendre l'intensité de la lumière laser plus faible dans une première partie comprenant un centre d'une section transversale de la lumière laser qui croise le chemin optique, rendre l'intensité de la lumière laser plus grande dans une deuxième partie de la lumière laser qui entoure la première partie dans la section transversale, et agrandir un diamètre de faisceau de la lumière laser, et
un écran (21) qui croise un chemin optique (27) de la lumière laser (23) ayant traversé l'élément de diffusion de sorte qu'une tache de lumière ayant traversé l'écran ou ayant été réfléchie par l'écran apparaisse sur une surface de l'écran, le diamètre de faisceau (f2) de la tache formant une taille de source lumineuse apparente du dispositif laser,
l'élément de diffusion étant configuré de sorte que la lumière laser se propage selon un premier angle de propagation substantiel lorsque la lumière laser traverse l'élément de diffusion, l'écran étant configuré de sorte que la lumière laser se propage selon un deuxième angle de propagation substantiel lorsque la lumière laser traverse l'écran ou est réfléchie par l'écran, et le premier angle de propagation substantiel étant supérieur au deuxième angle de propagation substantiel,
le dispositif laser étant configuré pour irradier un objet physique (5) avec la lumière laser (8) ayant traversé l'écran ou la lumière laser ayant été réfléchie par l'écran ;
un photodétecteur (2) qui est configuré pour détecter au moins une partie de la lumière laser (11) renvoyée par l'objet physique et délivrer en sortie un signal électrique (15) ; et
un circuit de commande (7) qui est configuré pour commander le dispositif laser et le photodétecteur,
dans lequel le circuit de commande amène le dispositif laser à irradier l'objet physique avec au moins une impulsion optique (38) de la lumière laser et amène le photodétecteur à effectuer une mesure résolue dans le temps d'au moins une impulsion optique réfléchie (19) de la lumière laser renvoyée par l'objet physique.

2. Appareil de détection optique selon la revendication 1, dans lequel un diamètre de faisceau de la lumière laser sur la surface de diffusion est inférieur à 10 mm.

3. Appareil de détection optique selon la revendication 1 ou 2, dans lequel un diamètre de faisceau de la lumière laser sur l'écran n'est pas inférieur à 10 mm.

4. Appareil de détection optique selon l'une des revendications 1 à 3, dans lequel un diamètre de faisceau de la lumière laser sur une surface de l'objet physique est supérieur à un diamètre de faisceau de la lumière laser sur l'écran.

5. Appareil de détection optique selon l'une des revendications 1 à 4, dans lequel l'élément de diffusion comprend en outre un réseau de lentilles sur la surface de diffusion.

6. Appareil de détection optique selon l'une des revendications 1 à 5, dans lequel l'écran comprend une pluralité de dépressions et une pluralité de saillies agencées en alternance et de manière bidimensionnelle sur une surface de l'écran, et
une profondeur de chacune de la pluralité de dépressions et une hauteur de chacune de la pluralité de saillies se trouvent chacune dans une plage allant de 2 µm à 30 µm.

7. Appareil de détection optique selon l'une des revendications 1 à 5, dans lequel l'écran comprend une première couche et une deuxième couche, et
un indice de réfraction de la première couche est différent d'un indice de réfraction de la deuxième couche.

8. Appareil de détection optique selon l'une des revendications 1 à 5, dans lequel l'écran comprend une pluralité de premières parties et une pluralité de deuxièmes parties agencées en alternance et de manière bidimensionnelle sur une surface de l'écran, et
un indice de réfraction de chacune de la pluralité de premières parties est différent d'un indice de réfraction de chacune de la pluralité de deuxièmes parties.

9. Appareil de détection optique selon l'une des revendications 1 à 8, dans lequel 1/µs ≤ d ≤ 1/µs', où d est l'épaisseur de l'écran, µs' est le coefficient de diffusion équivalent de l'écran, et µs est le coefficient de diffusion de l'écran.

10. Appareil de détection optique selon l'une des revendications 1 à 9, dans lequel une distance de l'élément de diffusion à l'écran est plus longue qu'une distance de la source de lumière à l'élément de diffusion.

11. Appareil de détection optique selon l'une des revendications 1 à 10, comprenant en outre un élément optique, disposé entre l'élément de diffusion et l'écran, qui a une première surface et une deuxième surface opposée à la première surface,
dans lequel l'élément de diffusion est disposé sur la première surface, et
l'écran est disposé sur la deuxième surface.

12. Appareil de détection optique selon l'une des revendications 1 à 10, comprenant en outre :
un premier élément optique ayant une première surface et une deuxième surface opposée à la première surface ; et
un deuxième élément optique ayant une troisième surface et une quatrième surface opposée à la troisième surface,
dans lequel le premier élément optique et le deuxième élément optique sont tous deux disposés entre l'élément de diffusion et l'écran,
l'élément de diffusion est disposé sur la première surface,
l'écran est disposé sur la quatrième surface, et
la deuxième surface fait face à la troisième surface.

13. Appareil de détection optique selon l'une des revendications 1 à 10, comprenant en outre :
un premier élément optique ayant une première surface et une deuxième surface opposée à la première surface ; et
un deuxième élément optique ayant une troisième surface et une quatrième surface opposée à la troisième surface,
dans lequel le premier élément optique est disposé entre l'élément de diffusion et l'écran,
l'élément de diffusion est disposé sur la première surface,
l'écran est disposé sur la troisième surface,
la deuxième surface fait face à une surface de l'écran,
la deuxième surface est inclinée par rapport à la surface de l'écran, et
le dispositif laser irradie l'objet physique avec la lumière laser réfléchie par la surface de l'écran.

14. Appareil de détection optique selon l'une des revendications 1 à 13, comprenant en outre une lentille collimatrice située entre la source de lumière et l'élément de diffusion.

15. Appareil de détection optique selon l'une des revendications 1 à 14, dans lequel le circuit de commande amène le photodétecteur à détecter une composante de lumière incluse dans une période de chute de l'au moins une impulsion optique réfléchie et à délivrer en sortie un signal électrique représentant une quantité de la composante, et
la période de chute est une période allant du début à la fin d'une diminution d'intensité de l'au moins une impulsion optique réfléchie.

16. Appareil de détection optique selon la revendication 15, dans lequel le signal électrique contient des informations sur un état interne de l'objet physique.

17. Appareil de détection optique selon la revendication 16, dans lequel l'objet physique est un corps vivant, et
le signal électrique contient des informations sur le flux sanguin dans le corps vivant.

18. Appareil de détection optique selon la revendication 16, dans lequel l'objet physique est le front d'un humain, et
le signal électrique contient des informations sur le flux sanguin cérébral.

19. Appareil de détection optique selon l'une des revendications 1 à 18, dans lequel le signal électrique contient des informations sur une distance entre l'objet physique et le photodétecteur.

20. Appareil de détection optique selon la revendication 19, dans lequel le photodétecteur est une caméra à temps de vol.
